(19) Europäisches Patentamt
European Patent Office
Office européen des brevets

(11) **EP 4 613 779 A1**

(12) **EUROPEAN PATENT APPLICATION**
published in accordance with Art. 153(4) EPC

(43) Date of publication:
**10.09.2025 Bulletin 2025/37**

(21) Application number: 23884813.9

(22) Date of filing: **30.10.2023**

(51) International Patent Classification (IPC):
*C07K 19/00* (2006.01)        *C12N 15/13* (2006.01)
*C12N 15/62* (2006.01)        *C12N 5/10* (2006.01)
*A61K 35/17* (2025.01)        *A61P 35/00* (2006.01)

(52) Cooperative Patent Classification (CPC):
**A61K 35/17; A61P 35/00; C07K 16/00;
C07K 19/00; C12N 5/10; C12N 15/62**

(86) International application number:
**PCT/CN2023/127598**

(87) International publication number:
**WO 2024/093882 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.11.2022  PCT/CN2022/128954**

(71) Applicants:
• **Acroimmune Biopharma Co., Ltd.
Nanjing, Jiangsu 211100 (CN)**
• **Acroimmune Biotech Co., Ltd.
Guangzhou, Guangdong 510005 (CN)**

(72) Inventors:
• **TONG, Dan
Nanjing, Jiangsu 211100 (CN)**
• **LIU, Yang
Nanjing, Jiangsu 211100 (CN)**

(74) Representative: **Weickmann & Weickmann
PartmbB
Postfach 860 820
81635 München (DE)**

Remarks:
The complete document including Reference
Table(s) and the Sequence Listing(s) can be
downloaded from the EPO website

(54) **CD24 BINDING PROTEIN AND USE THEREOF**

(57)    Provided are a group CD24 binding proteins and uses thereof. Specifically provided is a group of antigen binding proteins, wherein cells expressing the antigen binding protein can have cytotoxicity and safety. Also provided is a use of the antigen binding protein in the preparation of a medication.

**Description**

**FIELD OF THE INVENTION**

The present application relates to the field of biological medicine, and in particular to a CD24-binding protein and uses thereof.

**BACKGROUND OF THE INVENTION**

Immune cells expressing chimeric antigen receptors (CAR) can specifically recognize antigens expressed on the surface of tumor cells and mediated killing and removing tumors. For example, when antigen-positive cells targeted by a CAR are present, cells expressing the CAR can recognize and kill these antigen-positive cells.

However, the existing CAR in the art still has problems such as low activity, weak cytotoxic efficacy of CAR-expressing cells, and problematic *in vivo* safety. Therefore, it is desirable to further optimize the CAR in the art, in order to achieve the effect of increasing the cytotoxic efficacy of CAR-expressing cells and improve safety.

**SUMMARY OF THE INVENTION**

The present application provides an antigen-binding protein with an improved structure, and the antigen-binding protein may be in the form of a chimeric antigen receptor (CAR). The antigen-binding protein may have one or more effects selected from the group consisting of: (1) excellent expression efficiency; (2) significant antitumor activity of cells expressing the antigen-binding protein; (3) sustained antitumor activity of cells expressing the antigen-binding protein; (4) significant cell activation ability of cells expressing the antigen-binding protein; and (5) appropriate cytokine secretion capacity of cells expressing the antigen-binding protein.

In one aspect, the present application provides an antigen-binding protein, wherein the antigen-binding protein binds to CD24, after which cells expressing the antigen-binding protein have an ability to express cytokines at a low level. For example, the antigen-binding fragment comprises a light chain variable region and a heavy chain variable region, with a linker between the light chain variable region and the heavy chain variable region, the linker having a length of 10 amino acids or less.

In another aspect, the present application provides a polypeptide, comprising the antigen-binding protein of the present application.

In another aspect, the present application provides a nucleic acid, encoding the antigen-binding protein of the present application and/or the polypeptide of the present application.

In another aspect, the present application provides a vector, comprising the nucleic acid of the present application.

In another aspect, the present application provides a cell, comprising the antigen-binding protein of the present application, the polypeptide of the present application, the nucleic acid of the present application and/or the vector of the present application.

In another aspect, the present application provides a preparation method for the antigen-binding protein of the present application and/or the polypeptide of the present application, wherein the preparation method comprises culturing the cell of the present application under conditions allowing expression of the antigen-binding protein and/or the polypeptide.

In another aspect, the present application provides a composition, comprising the antigen-binding protein of the present application, the polypeptide of the present application, the nucleic acid of the present application, the vector of the present application, and/or the cell of the present application, and optionally, a pharmaceutically acceptable carrier.

In another aspect, the present application provides a kit, comprising the antigen-binding protein of the present application, the polypeptide of the present application, the nucleic acid of the present application, the vector of the present application, the cell of the present application, and/or the composition of the present application.

In another aspect, the present application provides a method for stimulating an immune response, comprising administering the antigen-binding protein of the present application, the polypeptide of the present application, the nucleic acid of the present application, the vector of the present application, the cell of the present application, the composition of the present application and/or the kit of the present application.

In another aspect, the present application provides a use of the antigen-binding protein of the present application, the polypeptide of the present application, the nucleic acid of the present application, the vector of the present application, the cell of the present application, the composition of the present application and/or the kit of the present application in preparation of a medicament, said medicament is used for preventing, alleviating and/or treating a tumor.

In another aspect, the present application provides a method for preventing, alleviating and/or treating a tumor, comprising administering the antigen-binding protein of the present application, the polypeptide of the present application, the nucleic acid of the present application, the vector of the present application, the cell of the present application, the composition of the present application and/or the kit of the present application.

In another aspect, the present application provides a use of the antigen-binding protein of the present application, the polypeptide of the present application, the nucleic acid of the present application, the vector of the present application, the cell of the present application, the composition of the present application and/or the kit of the present application for preventing, alleviating and/or treating a tumor.

Other aspects and advantages of the present application can be readily perceived by those skilled in the art from the detailed description below. The detailed description below only illustrates and describes the exemplary embodiments of the present application. As would be appreciated by those skilled in the art, the content of the present application enables those killed in the art to change the disclosed specific embodiments without departing from the spirit and scope of the invention involved in the present application. Accordingly, the accompanying drawings and the description in the specification of the present application are merely for an exemplary but not restrictive purpose.

## BRIEF DESCRIPTION OF THE DRAWINGS

The specific features of the invention involved in the present application are listed in the appended claims. The characteristics and advantages of the invention involved in the present application can be better understood by referring to the exemplary embodiments and the accompanying drawings described in detail below. The accompanying drawings are briefly illustrated as follows.

FIG. 1 shows the antitumor activity and cytokine expression levels of H3L3-CAR and PP6373-CAR. C1 was derived by modifying the sequence of H3L3-CAR, C2 was derived by modifying the sequence of PP6373-CAR, and the modified C1 and C2 showed higher anti-tumor activity and cytokine secretion level.

FIG. 2 shows the expression of CAR structures such as C3, C5, C7, C9, C11, C13 and C15; all of these CARs were derived by adjusting the hinge region, transmembrane region and costimulatory domain of the CAR structure on the basis of C1.

FIG. 3 shows the antitumor activity of CAR structures such as C1, C3, C5, C7, C9, C11, C13 and C15 of the present application.

FIG. 4 shows the cytokine secretion of CAR structures such as C1, C3, C5, C7, C9, C11, C13 and C15 of the present application.

FIG. 5 shows the expression of CAR structures such as C4, C6, C8, C10, C12, C14 and C16, all derived by adjusting the hinge region, transmembrane region and costimulatory domain of the CAR structure on the basis of C2.

FIG. 6 shows the antitumor activity of CAR structures such as C2, C4, C6, C8, C10, C12, C14, and C16 of the present application.

FIG. 7 shows the cytokine secretion of CAR structures such as C2, C4, C6, C8, C10, C12, C14 and C16 of the present application.

FIG. 8 shows the expression of C23, C27 and C28, all derived by adjusting the length of a linker or the type of a transmembrane region of the present application.

FIG. 9 shows the *in vitro* cytotoxic efficacy of C23, C27, and C28, all derived by adjusting the length of a linker or the type of a transmembrane region of the present application.

FIG. 10 shows the cytokine secretion of C23, C27, and C28, all derived by adjusting the length of a linker or the type of a transmembrane region of the present application.

FIG. 11 shows the expression of C28, C29 and C30, all derived by adjusting the length of a linker of the present application.

FIG. 12 shows the *in vitro* cytotoxic efficacy and cytokine secretion of C28, C29, and C30 derived by adjusting the length of a linker of the present application.

FIG. 13 shows the expression of C31 derived by adding a chemokine receptor CXCR2 of the present application.

FIG. 14 shows the *in vitro* killing efficacy of C31 derived by adding a chemokine receptor CXCR2 of the present application.

FIG. 15 shows the cytokine secretion of C31 derived by adding a chemokine receptor CXCR2 of the present application.

FIG. 16 shows the *in vitro* cytotoxic efficacy and cytokine secretion of C32 derived by adding a cytokine IL-12β (p40) of the present application.

FIG. 17 shows the capacity of the CAR structures of C8 and C28 of the present application to clear tumors and their safety results.

FIG. 18 shows the capacity of the CAR structures of C28 and C31 of the present application to reach a tumor site.

FIG. 19 shows the capacity of the CAR structures of C28 and C31 of the present application to remove tumors.

FIG. 20 shows the capacity of the CAR structures of C28, C31 and C32 of the present application to remove tumors and the statistics of their safety results.

## DETAILED DESCRIPTION OF THE PREFERRED EMBODIMENTS

The embodiments of the invention of the present application will be illustrated by specific examples below. Those familiar with this technology can readily understand other advantages and effects of the invention of the present application from the content disclosed in the specification.

In the present application, the term "CD24" generally refers to a CD24 protein or a gene encoding the same. CD24 can be found under UniProt Number P25063. This term comprises its variants, homologs, and functionally active fragments.

In the present application, the term "antigen-binding protein" generally refers to a protein capable of binding to one or more antigens, or functionally active fragments thereof. For example, an exemplary antigen-binding protein comprises a chimeric antigen receptor and a variety of other molecules that are known in the art and comprise domains capable of recognizing antigens.

In the present application, the term "chimeric antigen receptor (CAR)" generally refers to a fusion protein comprising an extracellular domain capable of binding to an antigen, and at least one transmembrane domain. CAR can be the core component of a CAR cell and can comprise an antigen (e.g., a tumor-specific and/or tumor-associated antigen)-binding domain, a hinge domain, a transmembrane domain and an intracellular domain.

In the present application, the term "signal peptide" generally refers to a propeptide that is present as an N-terminal peptide on the precursor form of a protein. The signal peptide functions to facilitate the translocation of an expression polypeptide attached to the endoplasmic reticulum and to express the protein of interest on the cell membrane, during which the signal peptide can generally be cleaved. For example, without the signal peptide, the biological activity of the antigen-binding protein of the present application is not affected as well. For an organism for producing polypeptides, the signal peptide can be heterologous or homologous.

In the present application, the term "antigen-binding domain" generally refers to (specifically) binding to a given target epitope or site on a target molecule (antigen), or interacting with the given target epitope or site, or identifying a domain of the given target epitope or site.

In the present application, the term "specific binding" generally refers to a measurable and/or reproducible interaction, such as the binding between a target and an antibody, and the existence of the target can be determined in a heterogeneous population of molecules (including biomolecules). For example, an antibody specifically binding to a target (which may be an epitope) is an antibody that binds to the target with greater affinity, avidity, easiness, and/or duration than it binds to other targets. In some embodiments, the antibody specifically binds to an epitope on a protein, the epitope being conserved among proteins of different species. In another embodiment, the specific binding may include, but not necessarily, exclusive binding.

In the present application, the term "antibody" generally refers to an immunoglobulin or a fragment or derivative thereof, and encompasses any polypeptide, regardless of *in vitro* or *in vivo,* that includes an antigen-binding site. This term includes, but is not limited to, polyclonal, monoclonal, monospecific, multispecific, non-specific, humanized, single-chain, chimeric, synthetic, recombinant, hybrid, mutant and grafted antibodies. The term "antibody" may also comprise antibody fragments, such as Fab, F(ab')$_2$, Fv, scFv, Fd, dAb, and other antibody fragments that maintain the antigen-binding function.

In the present application, the term "single-chain antibody (scFv)" may be an antibody formed by linking the heavy chain variable region and the light chain variable region of the antibody via a linker.

In the present application, the term "hinge domain" generally refers to a linkage region between the antigen-binding domain and the transmembrane region. In general, the hinge domain has some degree of flexibility, and the CAR molecule may optionally comprise or not comprise the hinge domain.

In the present application, the term "transmembrane domain" generally refers to the domain in the CAR that crosses the cell membrane, links to the intracellular signaling domain and serves to transmit signals.

In the present application, the term "CD8a" generally refers to the T-cell surface glycoprotein CD8 alpha chain. CD8a can be found under UniProt Number P01732. This term comprises its variants, homologs, and functionally active fragments. In the present application, the term "CD28" generally refers to T-cell-specific surface glycoprotein CD28. CD28 can be found under UniProt Number P10747. This term comprises its variants, homologs, and functionally active fragments.

In the present application, the term "intracellular domain" generally refers to the intracellular fraction of a molecule. The intracellular domain produces signals that promote the immune effector functions of CAR-containing cells. For example, cell lysis activity and auxiliary activity are involved. For example, the intracellular domain may comprise only a truncated fraction that preserves the intracellular signaling function.

In the present application, the term "costimulatory signaling domain" generally refers to an intracellular domain that can provide immune costimulatory molecules. In the present application, the term "costimulatory" generally refers to the source of lymphocyte activation signals, which are typically produced by the interaction of costimulatory molecules and their receptors on the surface of immune cells involved in adaptive immunity (between T cells and B cells or between antigen-presenting cells and T cells).

In the present application, the term "signaling domain" generally refers to a domain that is located inside a cell and capable of conducting signals. In the present application, the intracellular signaling domain may transmit signals into a cell.

In the present application, the term "CD3ζ" generally refers to CD247 or T-cell surface glycoprotein CD3 zeta chain. CD3ζ can be found under UniProt Number P20963. This term comprises its variants, homologs, and functionally active fragments.

For example, the sequence mentioned in the present application comprises a sequence that is at least approximately 95% homologous to this sequence. For example, the sequence mentioned in the present application comprises a sequence that is at least approximately 95%, 96%, 97%, 98% or 99% homologous to this sequence.

In the present application, the term "immune cells" generally refers to cells involved in an immune response, for example, those promoting an immune effector response. Examples of the immune cells comprise, but are not limited to, T cells, B cells, natural killer (NK) cells, NKT cells, mast cells, granulocytes, monocytes, lymphocytes, and macrophages. This term also includes engineered immune cells, such as immune cells that have been genetically modified by adding exogenous genetic materials in the form of DNA or RNA to total genetic materials of the cells.

In the present application, the term "cytotoxic efficacy" refers to the killing of cells by exposing these cells to an effective amount of antibodies, immunoconjugates, bispecific/multispecific molecules, or their combinations. The method may comprise killing antigen expression-positive cells, optionally in the presence of effector cells, for example, by CDC, apoptosis, ADCC, ADCP, phagocytosis, or by a combination of two or more of these mechanisms.

In the present application, the term "directly or indirectly linked to" refers to direct linkage by peptide bonds, or indirect linkage by linkers or by non-peptide linkage.

In the present application, the term "proliferation" refers to an increase in cell division (symmetrical or asymmetric division of cells). "Proliferation" may refer to the symmetrical or asymmetric division of T cells. An "increase in proliferation" occurs when the count of cells in a treated sample increases as compared with cells in an untreated sample.

In the present application, the term "subject" includes any human or non-human animal. The term "non-human animals" comprise all vertebrates, for example, mammals and non-mammals, such as non-human primates, sheep, dogs, cats, cows, horses, chickens, amphibians and reptiles. The non-human animals may be mammals, such as non-human primates, sheep, dogs, cats, cows and horses.

In the present application, the term "therapeutically effective dose" refers to the dose of the antibody of the present application that is sufficient to prevent or alleviate symptoms associated with a disease or disorder (e.g., cancer). The therapeutically effective dose is associated with the disease being treated, and those skilled in the art can easily determine the actual effective dose.

In the present application, the term "medicament" generally refers to a chemical compound or composition that is capable of inducing the desired therapeutic effect when administered correctly to a patient.

In the present application, the term "composition" refers to a mixture comprising one or more of the compounds of the present application, or a physiologically/pharmaceutically acceptable salt or prodrug thereof, and other chemical components, for example physiological/pharmaceutically acceptable carriers and excipients. The pharmaceutical composition is intended to promote the administration to an organism, facilitating the absorption of an active ingredient to thereby exert a biological activity. A therapeutic composition should generally be sterile and remain stable under manufacturing and storage conditions. The composition may be formulated as a solution, a microemulsion, a dispersant, a liposome, or other ordered structures suitable for a high antibody concentration. A sterile injectable solution may be prepared by incorporating a required amount of active compound (i.e., the antibody or antibody moiety) together with one or a combination of the components listed above into an appropriate solvent, followed by filtration and sterilization as required.

In the present application, the term "vector" generally refers to a nucleic acid molecule capable of transferring another nucleic acid to which it is attached. One type of vector is a "plasmid", which refers to a circular double-stranded DNA loop into which other DNA segments can be linked. Another type of vector is a viral vector, in which other DNA segments may be linked into a viral genome. Some vectors are capable of autonomously replicating in host cells into which they are introduced into (e.g., bacterial vectors with origins of bacterial replication and episomal mammalian vectors). Other vectors (e.g., non-episomal mammalian vectors) may be integrated into the genomes of host cells when introduced into the host cells and thus replicate together with the host genomes. These vectors include, for example, naked RNA polynucleotides incapable of replicating autonomously, naked DNA polynucleotides, polynucleotides composed of DNA and RNA in the same strand, poly-lysine-conjugated DNA or RNA, peptide-conjugated DNA or RNA, liposome-conjugated DNA, etc. Furthermore, some vectors are capable of directing the expression of genes to which these vectors are effectively linked. Such vectors are referred to as "recombinant expression vectors" (or simply "expression vectors") in the present application. In general, the expression vectors used in recombinant DNA technologies are usually in the form of plasmids. In the present specification, the terms "plasmid" and "vector" are used interchangeably, as the plasmid is the most commonly used form of vector.

In the present application, the term "adjuvant" generally refers to any substance that assists or regulates the action of a medicament. It includes, but not is limited to, an immunological adjuvant, which enhances or diversifies the immune response to an antigen.

In the present application, the term "tumor" or "tumor cell" generally refers to or describes a physiological condition in mammals that is usually characterized by unregulated cell growth. Examples of tumors include, but are not limited to, carcinomas, lymphomas, blastomas (including medulloblastoma and retinoblastoma), sarcomas (including liposarcoma and synovial cell sarcoma), neuroendocrine tumors (including carcinoid tumors, gastrinomas, and islet-cell carcinomas), mesotheliomas, schwannomas (including acoustic neuromas), meningiomas, adenocarcinomas, and melanomas. The "tumor cell" may further include a "solid tumor", which refers to a tumor selected from the group consisting of: gastrointestinal cancer, pancreatic cancer, glioblastoma, cervical cancer, ovarian cancer, bladder cancer, hepatoma, breast cancer, colon cancer, rectal cancer, colorectal cancer, endometrial or uterine cancer, salivary gland cancer, kidney cancer, prostate cancer, vulvar cancer, thyroid cancer, hepatic carcinoma, anal cancer, penile cancer, testicular cancer, esophageal cancer, bile duct tumor, and head and neck cancer.

In the present application, the term "antigen-positive tumor" refers to tumor cells that express antigenic proteins on their surface. For the purpose of determining whether cells express antigenic proteins on their surface, antigen mRNA expression is considered to be related to antigenic protein expression on the cell surface, and the antigen mRNA expression may be determined by a method selected from in situ hybridization and RT-PCR (including quantitative RT-PCR). Or, for example, antibodies against antigenic proteins may be used to determine the antigenic protein expression on the cell surface in methods such as immunohistochemistry and FACS. For example, an antigen-positive tumor may be a mammalian implanted tumor. In the present application, the term "antigen-positive tumor cell" refers to a cell expressing antigenic proteins on it surface.

**DETAILED DESCRIPTION OF THE INVENTION**

In one aspect, the present application provides a group of improved antigen-binding protein G1, wherein the G1 binds to CD24, and the enhanced effect of cells expressing said G1 on the killing ability of CD24-positive tumor cells, or the ability of cells expressing said G1 to express cytokines after being stimulated by CD24-positive tumor cells, is higher than that of cells expressing the existing CD24 antigen-binding proteins. For example, approximately 5%, 10%, 20%, 30%, 40%, 50%, 70%, 100%, 150%, 200%, 300%, 400%, 500%, 4000%, 5000% or 5000% higher than cells expressing CD24 antigen-binding proteins that have been disclosed in the art.

In one aspect, the present application provides a group of further improved antigen-binding protein G2 based on the G1, wherein the G2 binds to CD24, G2-expressing cells maintain a capacity to kill the CD24-positive tumor cells, and an ability of expressing cytokines after stimulation by the CD24-positive tumor cells is at least about 50% lower than that of cells expressing G1. For example, at least approximately 5%, 10%, 20%, 30%, 40% or 50% lower than G1-expressing cells.

In one aspect, the present application provides a group of further improved antigen-binding protein G3 based on the G1 or G2 for use in expression in cells, wherein the G3-expressing cells also express chemokine receptors. For example, the chemokine receptors may be chemokine receptors known in the art. For example, the chemokine receptors may comprise CXCR2 or a functional fragment thereof. For example, the CXCR2 may comprise an amino acid sequence as set forth in SEQ ID NO: 53.

In one aspect, the present application provides a group of further improved antigen-binding protein G3 based on the G1 or the G2 for use in expression in cells, wherein the G3-expressing cells also express cytokines. For example, the cytokines may comprise IL-12β or a functional fragment thereof. For example, the IL-12β may comprise an amino acid sequence as set forth in SEQ ID NO: 50. In one aspect, the present application provides an antigen-binding protein, wherein the antigen-binding protein binds to CD24, after which cells expressing the antigen-binding protein have an ability to express cytokines at a low level.

For example, the cytokines comprise, but are not limited to, IFN-γ, IL-6, TNF-α, and/or IL-2.

For example, in terms of the antigen-binding protein as described, compared with the corresponding cells that do not express the antigen-binding protein of the present application, the cells expressing the antigen-binding protein have a cytokine expression level of approximately 2000 pg/ml or less in the co-culture supernatant of these cells and tumor cells for about 24 h. For example, compared with the corresponding cells that do not express the antigen-binding protein of the present application, the cells expressing the antigen-binding protein have a cytokine expression level of approximately 2000 pg/ml or less, approximately 1000 pg/ml or less, approximately 500 pg/ml or less, approximately 200 pg/ml or less, approximately 100 pg/ml or less, or approximately 50 pg/ml or less in the co-culture supernatant of these cells and tumor cells for about 24 h.

For example, the antigen-binding protein comprises an antigen-binding domain. For example, the antigen-binding domain is capable of recognizing an antibody to the antigen or an antigen-binding fragment thereof. For example, the antigen-binding fragment comprises a single-chain antibody. For example, the antigen-binding fragment comprises a heavy chain variable region. For example, the heavy chain variable region comprises a sequence as set forth in SEQ ID NO: 23 or SEQ ID NO: 24. For example, the heavy chain variable region comprises HCDRs 1 to 3. For example, the heavy chain variable region comprises HCDRs 1 to 3 as set forth in SEQ ID Nos: 42 to 44. For example, the antigen-binding fragment comprises a light chain variable region. For example, the light chain variable region comprises a sequence as set forth in SEQ ID NO: 25 or SEQ ID NO: 26. For example, the light chain variable region comprises LCDRs 1 to 3. For example, the light chain variable region comprises LCDRs 1 to 3 as set forth in SEQ ID Nos: 45 to 47.

For example, the antigen-binding fragment comprises a light chain variable region and a heavy chain variable region. For example, the light chain variable region comprises a sequence as set forth in SEQ ID NO: 25 or SEQ ID NO: 26, and the heavy chain variable region comprises a sequence as set forth in SEQ ID NO: 23 or SEQ ID NO: 24. For example, a linker is comprised between the light chain variable region and the heavy chain variable region. For example, the linker has a length of 1, 2, 3, 4, 5, 6, 7, 8, 9, 10, or more amino acids. For example, the linker comprises an alanine-serine polymer or other flexible peptide sequences known in the art. For example, the linker comprises amino acid substitutions. For example, the linker comprises a sequence of 1 or more GGGGSs (as set forth in SEQ ID NO: 27), such as 1, 2, 3, 4, or 5 GGGGSs; for example, the linker comprises a sequence of 1 or more Gs (as set forth in SEQ ID NO: 28), such as 1, 2, 3, 4, 5, 6, 7, 8, 9, or 10 Gs; for example, the linker comprises a sequence of one or more GSs (SEQ ID NO: 29), such as 1, 2, 3, 4, or 5 GSs; for example, the linker comprises a sequence of one or more GGGSs (as set forth in SEQ ID NO: 30), such as 1, 2 or, 3, 4, or 5 GGGSs; and for example, the linker comprises a sequence of one or more GSGGSs (SEQ ID NO: 31), such as 1 or 2 GSGGSs.

For example, the antigen-binding protein further comprises a hinge domain, which is directly or indirectly linked to the antigen-binding domain. For example, the hinge domain comprises a hinge domain structure known in the art for chimeric antigen receptors. For example, the hinge domain comprises, but is not limited to, a hinge domain of CD8 or CD28, or of a functional fragment thereof. For example, the hinge domain comprises a sequence as set forth in SEQ ID NO: 32 or 33.

For example, the antigen-binding protein further comprises a transmembrane domain, which is directly or indirectly linked to the hinge domain. For example, the transmembrane domain of the present application comprises an intracellular domain structure known in the art for chimeric antigen receptors. For example, the transmembrane domain comprises, but is not limited to, a transmembrane domain of CD8 or CD28, or of a functional fragment thereof. For example, the transmembrane domain comprises a sequence as set forth in SEQ ID NO: 34 or 35.

For example, the antigen-binding protein further comprises an intracellular domain, which is directly or indirectly linked to the transmembrane domain. For example, the intracellular domain of the present application comprises an intracellular domain structure known in the art for chimeric antigen receptors. For example, the antigen-binding protein comprises one or more intracellular domains. For example, the antigen-binding protein comprises 1, 2, 3, 4, or 5 of the additional intracellular domains.

For example, the intracellular domain may comprise a costimulatory signal domain and/or a signaling domain. For example, the intracellular domain may comprise 1, 2, 3, 4, or 5 of the costimulatory signal domains. For example, the intracellular domain may comprise 1 of the costimulatory signal domains. For example, the costimulatory domain comprises, but is not limited to, the costimulatory domain of 41-BB, CD28, or OX40, or functional fragments thereof. For example, the costimulatory domain comprises a sequence as set forth in SEQ ID NO: 36, SEQ ID NO: 37 or SEQ ID NO: 38. For example, the costimulatory domain comprises a costimulatory domain of 41-BB, CD28 or a functional fragment thereof. For example, the costimulatory domain comprises sequences as set forth in SEQ ID NO: 36 and SEQ ID NO: 37. For example, the costimulatory domain comprises a costimulatory domain of 41-BB or a functional fragment thereof. For example, the costimulatory domain comprises a sequence as set forth in SEQ ID NO: 36. For example, the costimulatory domain comprises a costimulatory domain of CD28 or a functional fragment thereof. For example, the costimulatory domain comprises a sequence as set forth in SEQ ID NO: 37. For example, the costimulatory domain comprises a costimulatory domain of OX40 or a functional fragment thereof. For example, the costimulatory domain comprises a sequence as set forth in SEQ ID NO: 38. For example, the intracellular domain may comprise 1, 2, 3, 4, or 5 of the signaling domains. For example, the signaling domain comprises, but is not limited to, a signaling domain of CD3$\zeta$ or a functional fragment thereof. For example, the signaling domain comprises a sequence as set forth in SEQ ID NO: 39.

For example, the antigen-binding protein comprises a chimeric antigen receptor.

For example, the chimeric antigen receptor may comprise an antigen-binding domain, a hinge domain, a transmembrane domain, and/or an intracellular domain in sequence from the N-terminal to the C-terminal.

For example, the chimeric antigen receptor of the present application comprises an H3L3 heavy chain variable region as set forth in SEQ ID NO: 23, an H3L3 light chain variable region as set forth in SEQ ID NO: 25, a CD28 hinge domain as set forth in SEQ ID NO: 32, a CD28 transmembrane domain as set forth in SEQ ID NO: 34, a CD28 intracellular costimulatory domain as set forth in SEQ ID NO: 37, a 41-BB intracellular costimulatory domain as set forth in SEQ ID NO: 36, and a CD3$\zeta$ intracellular signaling domain as set forth in SEQ ID NO: 39.

For example, the chimeric antigen receptor of the present application comprises an H3L3 heavy chain variable region as set forth in SEQ ID NO: 23, an H3L3 light chain variable region as set forth in SEQ ID NO: 25, a CD8 hinge domain as set forth in SEQ ID NO: 33, a CD28 transmembrane domain as set forth in SEQ ID NO: 34, a CD28 intracellular costimulatory domain as set forth in SEQ ID NO: 37, a 41-BB intracellular costimulatory domain as set forth in SEQ ID NO: 36, and a CD3$\zeta$ intracellular signaling domain as set forth in SEQ ID NO: 39.

For example, the chimeric antigen receptor of the present application comprises an H3L3 heavy chain variable region as set forth in SEQ ID NO: 23, an H3L3 light chain variable region as set forth in SEQ ID NO: 25, a CD28 hinge domain as set forth in SEQ ID NO: 32, a CD28 transmembrane domain as set forth in SEQ ID NO: 34, a CD28 intracellular costimulatory domain as set forth in SEQ ID NO: 37, and a CD3$\zeta$ intracellular signaling domain as set forth in SEQ ID NO: 39.

For example, the chimeric antigen receptor of the present application comprises an H3L3 heavy chain variable region as set forth in SEQ ID NO: 23, an H3L3 light chain variable region as set forth in SEQ ID NO: 25, a CD8 hinge domain as set forth in SEQ ID NO: 33, a CD28 transmembrane domain as set forth in SEQ ID NO: 34, a CD28 intracellular costimulatory domain as set forth in SEQ ID NO: 37, and a CD3$\zeta$ intracellular signaling domain as set forth in SEQ ID NO: 39.

For example, the chimeric antigen receptor of the present application comprises an H3L3 heavy chain variable region as set forth in SEQ ID NO: 23, an H3L3 light chain variable region as set forth in SEQ ID NO: 25, a CD28 hinge domain as set forth in SEQ ID NO: 32, a CD28 transmembrane domain as set forth in SEQ ID NO: 34, a 41BB intracellular costimulatory domain as set forth in SEQ ID NO: 36, and a CD3$\zeta$ intracellular signaling domain as set forth in SEQ ID NO:39.

For example, the chimeric antigen receptor of the present application comprises an H3L3 heavy chain variable region as set forth in SEQ ID NO: 23, an H3L3 light chain variable region as set forth in SEQ ID NO: 25, a CD8 hinge domain as set forth in SEQ ID NO: 33, a CD28 transmembrane domain as set forth in SEQ ID NO: 34, a 41BB intracellular costimulatory domain as set forth in SEQ ID NO: 36, and a CD3$\zeta$ intracellular signaling domain as set forth in SEQ ID NO:39.

For example, the chimeric antigen receptor of the present application comprises an H3L3 heavy chain variable region as set forth in SEQ ID NO: 23, an H3L3 light chain variable region as set forth in SEQ ID NO: 25, a CD28 hinge domain as set forth in SEQ ID NO: 32, a CD28 transmembrane domain as set forth in SEQ ID NO: 34, an OX40 intracellular costimulatory domain as set forth in SEQ ID NO: 38, and a CD3$\zeta$ intracellular signaling domain as set forth in SEQ ID NO:39.

For example, the chimeric antigen receptor of the present application comprises an H3L3 heavy chain variable region as set forth in SEQ ID NO: 23, an H3L3 light chain variable region as set forth in SEQ ID NO: 25, a CD8 hinge domain as set forth in SEQ ID NO: 33, a CD28 transmembrane domain as set forth in SEQ ID NO: 34, an OX40 intracellular costimulatory domain as set forth in SEQ ID NO: 38, and a CD3$\zeta$ intracellular signaling domain as set forth in SEQ ID NO: 39.

For example, the chimeric antigen receptor of the present application comprises a PP6373 heavy chain variable region as set forth in SEQ ID NO: 24, a PP6373 light chain variable region as set forth in SEQ ID NO: 26, a CD28 hinge domain as set forth in SEQ ID NO: 32, a CD28 transmembrane domain as set forth in SEQ ID NO: 34, a CD28 intracellular costimulatory domain as set forth in SEQ ID NO: 37, a 41-BB intracellular costimulatory domain as set forth in SEQ ID NO: 36, and a CD3$\zeta$ intracellular signaling domain as set forth in SEQ ID NO: 39.

For example, the chimeric antigen receptor of the present application comprises a PP6373 heavy chain variable region as set forth in SEQ ID NO: 24, a PP6373 light chain variable region as set forth in SEQ ID NO: 26, a CD8 hinge domain as set forth in SEQ ID NO: 33, a CD28 transmembrane domain as set forth in SEQ ID NO: 34, a CD28 intracellular costimulatory domain as set forth in SEQ ID NO: 37, a 41-BB intracellular costimulatory domain as set forth in SEQ ID NO: 36, and a CD3ζ intracellular signaling domain as set forth in SEQ ID NO: 39.

For example, the chimeric antigen receptor of the present application comprises a PP6373 heavy chain variable region as set forth in SEQ ID NO: 24, a PP6373 light chain variable region as set forth in SEQ ID NO: 26, a CD28 hinge domain as set forth in SEQ ID NO: 32, a CD28 transmembrane domain as set forth in SEQ ID NO: 34, a CD28 intracellular costimulatory domain as set forth in SEQ ID NO: 37, and a CD3ζ intracellular signaling domain as set forth in SEQ ID NO: 39.

For example, the chimeric antigen receptor of the present application comprises a PP6373 heavy chain variable region as set forth in SEQ ID NO: 24, a PP6373 light chain variable region as set forth in SEQ ID NO: 26, a CD8 hinge domain as set forth in SEQ ID NO: 33, a CD28 transmembrane domain as set forth in SEQ ID NO: 34, a CD28 intracellular costimulatory domain as set forth in SEQ ID NO: 37, and a CD3ζ intracellular signaling domain as set forth in SEQ ID NO: 39.

For example, the chimeric antigen receptor of the present application comprises a PP6373 heavy chain variable region as set forth in SEQ ID NO: 24, a PP6373 light chain variable region as set forth in SEQ ID NO: 26, a CD28 hinge domain as set forth in SEQ ID NO: 32, a CD28 transmembrane domain as set forth in SEQ ID NO: 34, a 41-BB intracellular costimulatory domain as set forth in SEQ ID NO: 36, and a CD3ζ intracellular signaling domain as set forth in SEQ ID NO: 39.

For example, the chimeric antigen receptor of the present application comprises a PP6373 heavy chain variable region as set forth in SEQ ID NO: 24, a PP6373 light chain variable region as set forth in SEQ ID NO: 26, a CD8 hinge domain as set forth in SEQ ID NO: 33, a CD28 transmembrane domain as set forth in SEQ ID NO: 34, a 41-BB intracellular costimulatory domain as set forth in SEQ ID NO: 36, and a CD3ζ intracellular signaling domain as set forth in SEQ ID NO: 39.

For example, the chimeric antigen receptor of the present application comprises a PP6373 heavy chain variable region as set forth in SEQ ID NO: 24, a PP6373 light chain variable region as set forth in SEQ ID NO: 26, a CD28 hinge domain as set forth in SEQ ID NO: 32, a CD28 transmembrane domain as set forth in SEQ ID NO: 34, an OX40 intracellular costimulatory domain as set forth in SEQ ID NO: 38, and a CD3ζ intracellular signaling domain as set forth in SEQ ID NO:39.

For example, the chimeric antigen receptor of the present application comprises a PP6373 heavy chain variable region as set forth in SEQ ID NO: 24, a PP6373 light chain variable region as set forth in SEQ ID NO: 26, a CD8 hinge domain as set forth in SEQ ID NO: 33, a CD28 transmembrane domain as set forth in SEQ ID NO: 34, an OX40 intracellular costimulatory domain as set forth in SEQ ID NO: 38, and a CD3ζ intracellular signaling domain as set forth in SEQ ID NO:39.

For example, the chimeric antigen receptor of the present application may also be linked to a chemokine receptor via a self-cleaving peptide at the C-terminal of the CD3ζ intracellular signaling domain. For another example, the chimeric antigen receptor of the present application may also be linked to a cytokine via a self-cleaving peptide at the C-terminal of the CD3ζ intracellular signaling domain. For example, the linkage may be down via a 2A peptide. An exemplary 2A peptide may be P2A or T2A, with sequences as set forth in SEQ ID NO: 51 and SEQ ID NO: 52.

For example, the chemokine receptor may be CXCR2 or a functional fragment thereof. The exemplary amino acid sequence of CXCR2 may be as set forth in SEQ ID NO: 53.

For example, the cytokine may be IL-12β or a functional fragment thereof. The exemplary amino acid sequence of IL-12β may be as set forth in SEQ ID NO: 50.

For example, the improved antigen-binding protein of the present application comprises an amino acid sequence as set forth in any one of SEQ ID NOs: 1 to 21, 48 and 49.

For example, the improved antigen-binding protein of the present application comprises an amino acid sequence as set forth in SEQ ID NO: 1.

For example, the improved antigen-binding protein of the present application comprises an amino acid sequence as set forth in SEQ ID NO: 2.

For example, the improved antigen-binding protein of the present application comprises an amino acid sequence as set forth in SEQ ID NO: 3.

For example, the improved antigen-binding protein of the present application comprises an amino acid sequence as set forth in SEQ ID NO: 4.

For example, the improved antigen-binding protein of the present application comprises an amino acid sequence as set forth in SEQ ID NO: 5.

For example, the improved antigen-binding protein of the present application comprises an amino acid sequence as set forth in SEQ ID NO: 6.

For example, the improved antigen-binding protein of the present application comprises an amino acid sequence as set forth in SEQ ID NO: 7.

For example, the improved antigen-binding protein of the present application comprises an amino acid sequence as set forth in SEQ ID NO: 8.

For example, the improved antigen-binding protein of the present application comprises an amino acid sequence as set forth in SEQ ID NO: 9.

For example, the improved antigen-binding protein of the present application comprises an amino acid sequence as set forth in SEQ ID NO: 10.

For example, the improved antigen-binding protein of the present application comprises an amino acid sequence as set forth in SEQ ID NO: 11.

For example, the improved antigen-binding protein of the present application comprises an amino acid sequence as set forth in SEQ ID NO: 12.

For example, the improved antigen-binding protein of the present application comprises an amino acid sequence as set forth in SEQ ID NO: 13.

For example, the improved antigen-binding protein of the present application comprises an amino acid sequence as set forth in SEQ ID NO: 14.

For example, the improved antigen-binding protein of the present application comprises an amino acid sequence as set forth in SEQ ID NO: 15.

For example, the improved antigen-binding protein of the present application comprises an amino acid sequence as set forth in SEQ ID NO: 16.

For example, the improved antigen-binding protein of the present application comprises an amino acid sequence as set forth in SEQ ID NO: 17.

For example, the improved antigen-binding protein of the present application comprises an amino acid sequence as set forth in SEQ ID NO: 18.

For example, the improved antigen-binding protein of the present application comprises an amino acid sequence as set forth in SEQ ID NO: 19.

For example, the improved antigen-binding protein of the present application comprises an amino acid sequence as set forth in SEQ ID NO: 20.

For example, the improved antigen-binding protein of the present application comprises an amino acid sequence as set forth in SEQ ID NO: 21.

For example, the improved antigen-binding protein of the present application comprises an amino acid sequence as set forth in SEQ ID NO: 48.

For example, the improved antigen-binding protein of the present application comprises an amino acid sequence as set forth in SEQ ID NO: 49.

For example, the antigen-binding protein comprises a chimeric antigen receptor.

For example, the chimeric antigen receptor of the present application may be expressed on the surface of an immune cell. For example, the immune cell of the present application may comprise an immune effector cell. For example, the immune cell of the present application may comprise a T cell, a NK cell, and a mixture of the above cells. For example, the immune cell of the present application may comprise one or more chimeric antigen receptors of the present application.

In one aspect, the present application provides a polypeptide, which may comprise the antigen-binding protein of the present application. For example, the polypeptide of the present application may further comprise one or more types of full-length cytokines or fragments thereof.

In one aspect, the present application provides a nucleic acid, which may encode the antigen-binding protein of the present application and/or the polypeptide of the present application.

In one aspect, the present application provides a vector, which may comprise the nucleic acid of the present application.

In one aspect, the present application provides a cell, which may comprise the antigen-binding protein of the present application, the polypeptide of the present application, the nucleic acid of the present application, and/or the vector of the present application. For example, the cell may comprise an immune cell. For example, the cells may comprise a NK cell and/or a T cell.

In one aspect, the present application provides a preparation method for the antigen-binding protein of the present application and/or the polypeptide of the present application, wherein the preparation method may comprise culturing the cell of the present application under conditions allowing expression of the antigen-binding protein and/or the polypeptide.

In one aspect, the present application provides a composition, which may comprise the antigen-binding protein of the present application, the polypeptide of the present application, the nucleic acid of the present application, the vector of the present application, and/or the cell of the present application, and optionally a pharmaceutically acceptable carrier. For example, the pharmaceutically acceptable carrier may comprise any and all of solvents, dispersion media, isotonic agents, and absorption retarders, that are compatible with immune effector cells. Such carriers are generally safe and non-toxic.

In one aspect, the present application provides a kit, which may comprise the antigen-binding protein of the present application, the polypeptide of the present application, the nucleic acid of the present application, the vector of the present application, the cell of the present application, and/or the pharmaceutical composition of the present application.

In one aspect, the present application provides a method for stimulating an immune response, which may comprise administering the antigen-binding protein of the present application, the poly peptide of the present application, the nucleic acid of the present application, the vector of the present application, the cell of the present application, the pharmaceutical composition of the present application and/or the kit of the present application. For example, the method for stimulating an immune response may be *in vitro* and/or *ex vivo.* For example, the method for stimulating an immune response may be a method for a non-therapeutic purpose. In one aspect, stimulating the immune response may be increasing the capacity to kill target cells, decreasing the ability to release cytokines, and/or increasing the activation level of cells.

In one aspect, the present application provides a use of the antigen-binding protein of the present application, the polypeptide of the present application, the nucleic acid of the present application, the vector of the present application, the cell of the present application, the pharmaceutical composition of the present application and/or the kit of the present application in preparation of a medicament, said medicament is used for preventing, alleviating and/or treating a tumor. For example, the tumor may comprise solid tumor and/or hematologic tumor. For example, the tumor are selected from the group consisting of: gastric cancer, liver cancer, and leukemia. For example, the tumor of the present application may be selected from pan-cancer species, for example, the tumor may comprise a tumor that are positive to tumor antigens or have high expression of tumor antigens. For example, the tumor are selected from the group consisting of: lung cancer, breast cancer, liver cancer, brain cancer, cervical cancer, ovarian cancer, kidney cancer, testicular cancer, prostate cancer, and neuroblastoma.

In one aspect, the present application provides the antigen-binding protein of the present application, the polypeptide of the present application, the nucleic acid of the present application, the vector of the present application, the cell of the present application, the pharmaceutical composition of the present application and/or the kit of the present application, which can be used for preventing, alleviating and/or treating a tumor. For example, the tumor may comprise solid tumor and/or hematologic tumor. For example, the tumor are selected from the group consisting of: gastric cancer, liver cancer, and leukemia. For example, the tumor of the present application may be selected from pan-cancer species, for example, the tumor may comprise tumor that are positive to tumor antigens or have high expression of tumor antigens. For example, the tumor are selected from the group consisting of: lung cancer, breast cancer, liver cancer, brain cancer, cervical cancer, ovarian cancer, kidney cancer, testicular cancer, prostate cancer, and neuroblastoma.

In one aspect, the present application provides a method for preventing, alleviating and/or treating a tumor, comprising administering the antigen-binding protein of the present application, the polypeptide of the present application, the nucleic acid of the present application, the vector of the present application, the cell of the present application, the pharmaceutical composition of the present application and/or the kit of the present application. For example, the tumor may comprise solid tumor and/or hematologic tumor. For example, the tumor are selected from the group consisting of: gastric cancer, liver cancer, and leukemia. For example, the tumor of the present application may be selected from pan-cancer species, for example, the tumor may comprise a tumor that are positive to tumor antigens or have high expression of tumor antigens. For example, the tumor are selected from the group consisting of: lung cancer, breast cancer, liver cancer, brain cancer, cervical cancer, ovarian cancer, kidney cancer, testicular cancer, prostate cancer, and neuroblastoma.

In another aspect, the present application further provides embodiments as follows.

1. A group of improved antigen-binding protein G1, wherein the G1 is capable of binding to CD24, and the enhanced effect of cells expressing said G1 on the killing ability of CD24-positive tumor cells, or the ability of cells expressing said G1 to express cytokines after being stimulated by CD24-positive tumor cells, is higher than that of cells expressing the existing CD24 antigen-binding proteins.

2. The antigen-binding protein G1 of Embodiment 1, wherein the cytokines comprise IFN-γ, and/or IL-2.

3. A group of further improved antigen-binding protein G2 based on the G1 of any one of Embodiments 1 to 2, wherein the G2 is capable of binding to CD24, G2-expressing cells maintain a capacity to kill the CD24-positive tumor cells, and an ability of expressing cytokines after stimulation by the CD24-positive tumor cells is at least about 50% lower than that of cells expressing G1.

4. A group of further improved antigen-binding protein G3 based on the G1 of any one of Embodiments 1 to 2 or based on the G2 of Embodiment 3 for use in expression in cells, wherein the G3-expressing cells co-express chemokine receptors, and preferably, the chemokine receptors comprise CXCR2.

5. A group of further improved antigen-binding protein G3 based on the G1 of any one of Embodiments 1 to 2 or based on the G2 of Embodiment 3 for use in expression in cells, wherein the G3-expressing cells co-express cytokines, and preferably, the cytokines comprise IL-12β.

6. The antigen-binding protein of any one of Embodiments 1 to 5, wherein the antigen-binding fragment comprises a light chain variable region and a heavy chain variable region, with a linker between the light chain variable region and the heavy chain variable region, the linker having a length of 10 amino acids or less.

7. The antigen-binding protein of Embodiment 6, wherein the linker has a length of 5 amino acids or less.

8. The antigen-binding protein of any one of Embodiments 1 to 7, wherein said antigen-binding protein comprises H-CDRs 1 to 3 in said heavy chain variable region as set forth in SEQ ID NO: 23 or 24, and L-CDRs 1 to 3 in said light chain variable region as set forth in SEQ ID NO: 25 or 26.

9. The antigen-binding protein of any one of Embodiments 1 to 8, wherein the antigen-binding protein comprises H-CDRs 1 to 3 in the heavy chain variable region as set forth in SEQ ID NOs: 42 to 44, and L-CDRs 1 to 3 in the light chain variable region as set forth in SEQ ID NOs: 45 to 47.

10. The antigen-binding protein of any one of Embodiments 1 to 9, wherein the signal peptide comprises a sequence as set forth in SEQ ID NO: 22.

11. The antigen-binding protein of any one of Embodiments 1 to 10, wherein the antigen-binding protein further comprises a hinge domain, which is directly or indirectly linked to said antigen-binding domain.

12. The antigen-binding protein of Embodiment 11, wherein the hinge domain comprises a hinge domain of CD8 or CD28, or of a functional fragment thereof.

13. The antigen-binding protein of any one of Embodiments 11 to 12, wherein the hinge domain comprises a sequence as set forth in SEQ ID NO: 32 or 33.

14. The antigen-binding protein of any one of Embodiments 1 to 13, wherein the antigen-binding protein further comprises a transmembrane domain, which is directly or indirectly linked to the hinge domain.

15. The antigen-binding protein of Embodiment 14, wherein the transmembrane domain comprises a transmembrane domain of CD8 or CD28, or of a functional fragment thereof.

16. The antigen-binding protein of any one of Embodiments 14 to 15, wherein the transmembrane domain comprises a sequence as set forth in SEQ ID NO: 34 or 35.

17. The antigen-binding protein of any one of Embodiments 1 to 16, wherein the antigen-binding protein further comprises an intracellular domain, which is directly or indirectly linked to the transmembrane domain.

18. The antigen-binding protein of Embodiment 17, wherein the intracellular domain comprises a costimulatory domain, which comprises a costimulatory domain of 41-BB, CD28, or OX40, or of a functional fragment thereof.

19. The antigen-binding protein of Embodiment 18, wherein the antigen-binding protein comprises one or more of the costimulatory domains.

20. The antigen-binding protein of any one of Embodiments 18 to 19, wherein the costimulatory domain comprises a sequence as set forth in SEQ ID NO: 36, 37, or 38.

21. The antigen-binding protein of any one of Embodiments 1 to 20, wherein the intracellular domain comprises a signaling domain, which comprises a signaling domain of CD3ζ or a functional segment thereof.

22. The antigen-binding protein of Embodiment 21, wherein the intracellular signaling domain comprises a sequence as set forth in SEQ ID NO: 39.

23. The antigen-binding protein of any one of Embodiments 1 to 22, wherein the antigen-binding protein comprises a chimeric antigen receptor.

24. The antigen-binding protein of Embodiments 1 to 23, wherein the antigen-binding protein is selected from a sequence as set forth in any one of SEQ ID NOs: 1 to 21, 48 and 49.

25. A polypeptide, comprising the antigen-binding protein of any one of Embodiments 1 to 24.

26. The polypeptide of Embodiment 25, wherein the polypeptide further comprises a tagged protein, which comprises a sequence as set forth in SEQ ID NO: 40.

27. The polypeptide of Embodiment 26, wherein the tagged protein and the antigen-binding protein of the polypeptide are linked via a sequence as set forth in SEQ ID NO: 41.

28. A nucleic acid, encoding the antigen-binding protein of any one of Embodiments 1 to 24 and/or the polypeptide of any one of Embodiments 25 to 27.

29. A vector, comprising the nucleic acid of Embodiment 28.

30. A cell, comprising the antigen-binding protein of any one of Embodiments 1 to 24, the polypeptide of any one of Embodiments 25 to 27, the nucleic acid of Embodiment 28, and/or the vector of Embodiment 29.

31. The cell of Embodiment 30, wherein the cell comprises an immune cell.

32. The cell of any one of Embodiments 30 to 31, wherein the cell comprises an NK cell and/or a T cell.

33. A preparation method for the antigen-binding protein of any one of Embodiments 1 to 24 and/or the polypeptide of any one of Embodiments 25 to 27, wherein the preparation method comprises culturing the cell of any one of Embodiments 30 to 32 under conditions allowing expression of the antigen-binding protein and/or the polypeptide.

34. A composition, comprising the antigen-binding protein of any one of Embodiments 1 to 24, the polypeptide of any one of Embodiments 25 to 27, the nucleic acid of Embodiment 28, the vector of Embodiment 29, and/or the cell of any one of Embodiments 30 to 32, and optionally, a pharmaceutically acceptable carrier.

35. A kit, comprising the antigen-binding protein of any one of Embodiments 1 to 24, the polypeptide of any one of Embodiments 25 to 27, the nucleic acid of Embodiment 28, the vector of Embodiment 29, the cell of any one of Embodiments 30 to 32, and/or the composition of Embodiment 34.

36. A method for stimulating an immune response, comprising administering the antigen-binding protein of any one of Embodiments 1 to 24, the polypeptide of any one of Embodiments 25 to 27, the nucleic acid of Embodiment 28, the vector of Embodiment 29, the cell of any one of Embodiments 30 to 32, the composition of Embodiment 34 and/or the kit of Embodiment 35.

37. The use of the antigen-binding protein of any one of Embodiments 1 to 24, the polypeptide of any one of Embodiments 25 to 27, the nucleic acid of Embodiment 28, the vector of Embodiment 29, the cell of any one of Embodiments 30 to 32, the composition of Embodiment 34 and/or the kit of Embodiment 35 in preparation of a medicament, said medicament is used for preventing, alleviating, and/or treating a tumor.

38. The use of Embodiment 37, wherein said tumor comprise solid tumor and/or hematologic tumor.

39. The use of any one of Embodiments 37 to 38, wherein the tumors are selected from the group consisting of: lung cancer, breast cancer, liver cancer, brain cancer, cervical cancer, ovarian cancer, kidney cancer, testicular cancer, prostate cancer, and neuroblastoma.

Not wishing to be bound by any theory, the following examples are merely to illustrate the proteins, preparation methods, uses and the like of the present application, and are not intended to limit the scope of the invention of the present application.

**Examples**

| SEQ ID NO | Remark | Sequence |
|---|---|---|
| 1 | CAR-C1 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKRSVTVSSAAAIEVMYPPPYLDNEKSNGTIIHVKG KHLCPSPLFPGPSKPFWVLVVVGGVLACYSLLVTVAFIIFWVRSKRS RLLHSDYMNMTPRRPGPTRKHYQPYAPPRDFAAYRSKRGRKKLLY IFKQPFMRPVQTTQEEDGCSCRFPEEEEGGCELRVKFSRSADAPAY QQGQNQLYNELNLGRREEYDVLDKRRGRDPEMGGKPQRRKNPQE GLYNELQKDKMAEAYSEIGMKGERRRGKGHDGLYQGLSTATKDT YDALHMQALPPR |
| 2 | CAR-C2 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGSTGGGGSGGGGSGGGGSDI VMTQTPLSLSVTIGQPASISCKSSQSLLYSNGKTYLNWLQQRPGQSP KRLIYQVSKLDPGIPDRFSGSGSETDFTLKISRVEAEDLGIYYCMQG SSLPWTFGGGTKLEIKRSVTVSSAAAIEVMYPPPYLDNEKSNGTIIH VKGKHLCPSPLFPGPSKPFWVLVVVGGVLACYSLLVTVAFIIFWVR SKRSRLLHSDYMNMTPRRPGPTRKHYQPYAPPRDFAAYRSKRGRK KLLYIFKQPFMRPVQTTQEEDGCSCRFPEEEEGGCELRVKFSRSADA PAYQQGQNQLYNELNLGRREEYDVLDKRRGRDPEMGGKPQRRKN PQEGLYNELQKDKMAEAYSEIGMKGERRRGKGHDGLYQGLSTAT KDTYDALHMQALPPR |
| 3 | CAR-C3 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKASSHFVPVFLPAKPTTTPAPRPPTPAPTIASQPLS LRPEACRPAAGGAVHTRGLDFACDFWVLVVVGGVLACYSLLVTV<br><br>AFIIFWVRSKRSRLLHSDYMNMTPRRPGPTRKHYQPYAPPRDFAAY RSKRGRKKLLYIFKQPFMRPVQTTQEEDGCSCRFPEEEEGGCELRV KFSRSADAPAYQQGQNQLYNELNLGRREEYDVLDKRRGRDPEMG GKPQRRKNPQEGLYNELQKDKMAEAYSEIGMKGERRRGKGHDGL YQGLSTATKDTYDALHMQALPPR |

(continued)

| SEQ ID NO | Remark | Sequence |
|---|---|---|
| 4 | CAR-C4 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGSTGGGGSGGGGSGGGGSDI VMTQTPLSLSVTIGQPASISCKSSQSLLYSNGKTYLNWLQQRPGQSP KRLIYQVSKLDPGIPDRFSGSGSETDFTLKISRVEAEDLGIYYCMQG SSLPWTFGGGTKLEIKASSHFVPVFLPAKPTTTPAPRPPTPAPTIASQ PLSLRPEACRPAAGGAVHTRGLDFACDFWVLVVVGGVLACYSLLV TVAFIIFWVRSKRSRLLHSDYMNMTPRRPGPTRKHYQPYAPPRDFA AYRSKRGRKKLLYIFKQPFMRPVQTTQEEDGCSCRFPEEEEGGCEL RVKFSRSADAPAYQQGQNQLYNELNLGRREEYDVLDKRRGRDPE MGGKPQRRKNPQEGLYNELQKDKMAEAYSEIGMKGERRRGKGHD GLYQGLSTATKDTYDALHMQALPPR |
| 5 | CAR-C5 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKRSVTVSSAAAIEVMYPPPYLDNEKSNGTIIHVKG KHLCPSPLFPGPSKPFWVLVVVGGVLACYSLLVTVAFIIFWVRSKRS RLLHSDYMNMTPRRPGPTRKHYQPYAPPRDFAAYRSRVKFSRSAD APAYQQGQNQLYNELNLGRREEYDVLDKRRGRDPEMGGKPQRRK NPQEGLYNELQKDKMAEAYSEIGMKGERRRGKGHDGLYQGLSTA TKDTYDALHMQALPPR |
| 6 | CAR-C6 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGSTGGGGSGGGGSGGGGSDI VMTQTPLSLSVTIGQPASISCKSSQSLLYSNGKTYLNWLQQRPGQSP KRLIYQVSKLDPGIPDRFSGSGSETDFTLKISRVEAEDLGIYYCMQG SSLPWTFGGGTKLEIKRSVTVSSAAAIEVMYPPPYLDNEKSNGTIIH VKGKHLCPSPLFPGPSKPFWVLVVVGGVLACYSLLVTVAFIIFWVR SKRSRLLHSDYMNMTPRRPGPTRKHYQPYAPPRDFAAYRSRVKFS RSADAPAYQQGQNQLYNELNLGRREEYDVLDKRRGRDPEMGGKP QRRKNPQEGLYNELQKDKMAEAYSEIGMKGERRRGKGHDGLYQG LSTATKDTYDALHMQALPPR |
| 7 | CAR-C7 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKASSHFVPVFLPAKPTTTPAPRPPTPAPTIASQPLS LRPEACRPAAGGAVHTRGLDFACDFWVLVVVGGVLACYSLLVTV AFIIFWVRSKRSRLLHSDYMNMTPRRPGPTRKHYQPYAPPRDFAAY RSRVKFSRSADAPAYQQGQNQLYNELNLGRREEYDVLDKRRGRDP EMGGKPQRRKNPQEGLYNELQKDKMAEAYSEIGMKGERRRGKGH DGLYQGLSTATKDTYDALHMQALPPR |

(continued)

| SEQ ID NO | Remark | Sequence |
|---|---|---|
| 8 | CAR-C8 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGSTGGGGSGGGGSGGGGSDI VMTQTPLSLSVTIGQPASISCKSSQSLLYSNGKTYLNWLQQRPGQSP KRLIYQVSKLDPGIPDRFSGSGSETDFTLKISRVEAEDLGIYYCMQG<br><br>SSLPWTFGGGTKLEIKASSHFVPVFLPAKPTTTPAPRPPTPAPTIASQ PLSLRPEACRPAAGGAVHTRGLDFACDFWVLVVVGGVLACYSLLV TVAFIIFWVRSKRSRLLHSDYMNMTPRRPGPTRKHYQPYAPPRDFA AYRSRVKFSRSADAPAYQQGQNQLYNELNLGRREEYDVLDKRRG RDPEMGGKPQRRKNPQEGLYNELQKDKMAEAYSEIGMKGERRRG KGHDGLYQGLSTATKDTYDALHMQALPPR |
| 9 | CAR-C9 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKRSVTVSSAAAIEVMYPPPYLDNEKSNGTIIHVKG KHLCPSPLFPGPSKPFWVLVVVGGVLACYSLLVTVAFIIFWVKRGR KKLLYIFKQPFMRPVQTTQEEDGCSCRFPEEEEGGCELRVKFSRSAD APAYQQGQNQLYNELNLGRREEYDVLDKRRGRDPEMGGKPQRRK NPQEGLYNELQKDKMAEAYSEIGMKGERRRGKGHDGLYQGLSTA TKDTYDALHMQALPPR |
| 10 | CAR-C10 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGSTGGGGSGGGGSGGGGSDI VMTQTPLSLSVTIGQPASISCKSSQSLLYSNGKTYLNWLQQRPGQSP KRLIYQVSKLDPGIPDRFSGSGSETDFTLKISRVEAEDLGIYYCMQG SSLPWTFGGGTKLEIKRSVTVSSAAAIEVMYPPPYLDNEKSNGTIIH VKGKHLCPSPLFPGPSKPFWVLVVVGGVLACYSLLVTVAFIIFWVK RGRKKLLYIFKQPFMRPVQTTQEEDGCSCRFPEEEEGGCELRVKFS RSADAPAYQQGQNQLYNELNLGRREEYDVLDKRRGRDPEMGGKP QRRKNPQEGLYNELQKDKMAEAYSEIGMKGERRRGKGHDGLYQG LSTATKDTYDALHMQALPPR |
| 11 | CAR-C 11 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKASSHFVPVFLPAKPTTTPAPRPPTPAPTIASQPLS LRPEACRPAAGGAVHTRGLDFACDFWVLVVVGGVLACYSLLVTV AFIIFWVKRGRKKLLYIFKQPFMRPVQTTQEEDGCSCRFPEEEEGGC ELRVKFSRSADAPAYQQGQNQLYNELNLGRREEYDVLDKRRGRDP EMGGKPQRRKNPQEGLYNELQKDKMAEAYSEIGMKGERRRGKGH DGLYQGLSTATKDTYDALHMQALPPR |

| SEQ ID NO | Remark | Sequence |
|---|---|---|
| 12 | CAR-C12 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGSTGGGGSGGGGSGGGGSDI VMTQTPLSLSVTIGQPASISCKSSQSLLYSNGKTYLNWLQQRPGQSP KRLIYQVSKLDPGIPDRFSGSGSETDFTLKISRVEAEDLGIYYCMQG SSLPWTFGGGTKLEIKASSHFVPVFLPAKPTTTPAPRPPTPAPTIASQ PLSLRPEACRPAAGGAVHTRGLDFACDFWVLVVVGGVLACYSLLV TVAFIIFWVKRGRKKLLYIFKQPFMRPVQTTQEEDGCSCRFPEEEEG GCELRVKFSRSADAPAYQQGQNQLYNELNLGRREEYDVLDKRRG RDPEMGGKPQRRKNPQEGLYNELQKDKMAEAYSEIGMKGERRRG KGHDGLYQGLSTATKDTYDALHMQALPPR |
| 13 | CAR-C13 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKRSVTVSSAAAIEVMYPPPYLDNEKSNGTIIHVKG KHLCPSPLFPGPSKPFWVLVVVGGVLACYSLLVTVAFIIFWVRRDQ RLPPDAHKPPGGGSFRTPIQEEQADAHSTLAKIRVKFSRSADAPAYQ QGQNQLYNELNLGRREEYDVLDKRRGRDPEMGGKPQRRKNPQEG LYNELQKDKMAEAYSEIGMKGERRRGKGHDGLYQGLSTATKDTY DALHMQALPPR |
| 14 | CAR-C14 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGSTGGGGSGGGGSGGGGSDI VMTQTPLSLSVTIGQPASISCKSSQSLLYSNGKTYLNWLQQRPGQSP KRLIYQVSKLDPGIPDRFSGSGSETDFTLKISRVEAEDLGIYYCMQG SSLPWTFGGGTKLEIKRSVTVSSAAAIEVMYPPPYLDNEKSNGTIIH VKGKHLCPSPLFPGPSKPFWVLVVVGGVLACYSLLVTVAFIIFWVR RDQRLPPDAHKPPGGGSFRTPIQEEQADAHSTLAKIRVKFSRSADAP AYQQGQNQLYNELNLGRREEYDVLDKRRGRDPEMGGKPQRRKNP QEGLYNELQKDKMAEAYSEIGMKGERRRGKGHDGLYQGLSTATK DTYDALHMQALPPR |
| 15 | CAR-C15 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKASSHFVPVFLPAKPTTTPAPRPPTPAPTIASQPLS LRPEACRPAAGGAVHTRGLDFACDFWVLVVVGGVLACYSLLVTV AFIIFWVRRDQRLPPDAHKPPGGGSFRTPIQEEQADAHSTLAKIRVK FSRSADAPAYQQGQNQLYNELNLGRREEYDVLDKRRGRDPEMGG KPQRRKNPQEGLYNELQKDKMAEAYSEIGMKGERRRGKGHDGLY QGLSTATKDTYDALHMQALPPR |

(continued)

| SEQ ID NO | Remark | Sequence |
|---|---|---|
| 16 | CAR-C16 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGSTGGGGSGGGGSGGGGSDI VMTQTPLSLSVTIGQPASISCKSSQSLLYSNGKTYLNWLQQRPGQSP KRLIYQVSKLDPGIPDRFSGSGSETDFTLKISRVEAEDLGIYYCMQG SSLPWTFGGGTKLEIKASSHFVPVFLPAKPTTTPAPRPPTPAPTIASQ PLSLRPEACRPAAGGAVHTRGLDFACDFWVLVVVGGVLACYSLLV TVAFIIFWVRRDQRLPPDAHKPPGGGSFRTPIQEEQADAHSTLAKIR VKFSRSADAPAYQQGQNQLYNELNLGRREEYDVLDKRRGRDPEM GGKPQRRKNPQEGLYNELQKDKMAEAYSEIGMKGERRRGKGHDG LYQGLSTATKDTYDALHMQALPPR |
| 17 | CAR-C23 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSDIVM TQTPLSLSVTIGQPASISCKSSQSLLYSNGKTYLNWLLQKPGQSPKR LIYQVSKLDPGIPDRFSGSGSETDFTLKISRVEAEDVGIYYCMQGSSL PWTFGGGTKVEIKASSHFVPVFLPAKPTTTPAPRPPTPAPTIASQPLS LRPEACRPAAGGAVHTRGLDFACDIYIWAPLAGTCGVLLLSLVITL YCRSKRSRLLHSDYMNMTPRRPGPTRKHYQPYAPPRDFAAYRSRV KFSRSADAPAYQQGQNQLYNELNLGRREEYDVLDKRRGRDPEMG GKPQRRKNPQEGLYNELQKDKMAEAYSEIGMKGERRRGKGHDGL YQGLSTATKDTYDALHMQALPPR |
| 18 | CAR-C27 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGGGGSDIVMTQTPLSLSVTIG QPASISCKSSQSLLYSNGKTYLNWLQQRPGQSPKRLIYQVSKLDPGI PDRFSGSGSETDFTLKISRVEAEDLGIYYCMQGSSLPWTFGGGTKLE IKRSVTVSSAAAIEVMYPPPYLDNEKSNGTIIHVKGKHLCPSPLFPGP SKPFWVLVVVGGVLACYSLLVTVAFIIFWVRSKRSRLLHSDYMNM TPRRPGPTRKHYQPYAPPRDFAAYRSRVKFSRSADAPAYQQGQNQ LYNELNLGRREEYDVLDKRRGRDPEMGGKPQRRKNPQEGLYNEL QKDKMAEAYSEIGMKGERRRGKGHDGLYQGLSTATKDTYDALH MQALPPR |
| 19 | CAR-C28 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGGGGSDIVMTQTPLSLSVTIG QPASISCKSSQSLLYSNGKTYLNWLQQRPGQSPKRLIYQVSKLDPGI PDRFSGSGSETDFTLKISRVEAEDLGIYYCMQGSSLPWTFGGGTKLE IKASSHFVPVFLPAKPTTTPAPRPPTPAPTIASQPLSLRPEACRPAAG GAVHTRGLDFACDFWVLVVVGGVLACYSLLVTVAFIIFWVRSKRS RLLHSDYMNMTPRRPGPTRKHYQPYAPPRDFAAYRSRVKFSRSAD APAYQQGQNQLYNELNLGRREEYDVLDKRRGRDPEMGGKPQRRK NPQEGLYNELQKDKMAEAYSEIGMKGERRRGKGHDGLYQGLSTA TKDTYDALHMQALPPR |

(continued)

| SEQ ID NO | Remark | Sequence |
|---|---|---|
| 20 | CAR-C29 | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSSGGGGSGGGGSGGGGSGGGG SDIVMTQTPLSLSVTIGQPASISCKSSQSLLYSNGKTYLNWLLQKPG QSPKRLIYQVSKLDPGIPDRFSGSGSETDFTLKISRVEAEDVGIYYCM QGSSLPWTFGGGTKVEIKASSHFVPVFLPAKPTTTPAPRPPTPAPTIA SQPLSLRPEACRPAAGGAVHTRGLDFACDFWVLVVVGGVLACYSL LVTVAFIIFWVRSKRSRLLHSDYMNMTPRRPGPTRKHYQPYAPPRD FAAYRSRVKFSRSADAPAYQQGQNQLYNELNLGRREEYDVLDKRR GRDPEMGGKPQRRKNPQEGLYNELQKDKMAEAYSEIGMKGERRR GKGHDGLYQGLSTATKDTYDALHMQALPPR |
| 21 | CAR-C30 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGGGGSGGGGSDIVMTQTPLS LSVTIGQPASISCKSSQSLLYSNGKTYLNWLQQRPGQSPKRLIYQVS KLDPGIPDRFSGSGSETDFTLKISRVEAEDLGIYYCMQGSSLPWTFG GGTKLEIKASSHFVPVFLPAKPTTTPAPRPPTPAPTIASQPLSLRPEAC RPAAGGAVHTRGLDFACDFWVLVVVGGVLACYSLLVTVAFIIFWV RSKRSRLLHSDYMNMTPRRPGPTRKHYQPYAPPRDFAAYRSRVKF SRSADAPAYQQGQNQLYNELNLGRREEYDVLDKRRGRDPEMGGK PQRRKNPQEGLYNELQKDKMAEAYSEIGMKGERRRGKGHDGLYQ GLSTATKDTYDALHMQALPPR |
| 22 | CD8ss | MALPVTALLLPLALLLHAARP |
| 23 | H3L3 VH | EVQFVESGGGLVQPGGSLKLSCAASGVTFSEAWMDWVRQASGKG LEWVAEIRDKPNSYVTYYAESVKGRFTISRDDSKSTAYLQMNSLKT EDTAIYYCTGAMDYWGQGTLVTVSS |
| 24 | PP6373 VH | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSS |
| 25 | H3L3 VL | DIVMTQTPLSLSVTIGQPASISCKSSQSLLYSNGKTYLNWLLQKPGQ SPKRLIYQVSKLDPGIPDRFSGSGSETDFTLKISRVEAEDVGIYYCMQ GSSLPWTFGGGTKVEIK |
| 26 | PP6373 VL | DIVMTQTPLSLSVTIGQPASISCKSSQSLLYSNGKTYLNWLQQRPGQ SPKRLIYQVSKLDPGIPDRFSGSGSETDFTLKISRVEAEDLGIYYCMQ GSSLPWTFGGGTKLEIK |
| 27 | linker1 | (GGGGS)n, n is an integer selected from 1 to 5 |
| 28 | linker2 | (G)n, n is an integer selected from 1 to 10 |
| 29 | linker3 | (GS)n, n is an integer selected from 1 to 5 |
| 30 | linker4 | (GGGS)n, n is an integer selected from 1 to 5 |
| 31 | linker5 | (GSGGS)n, n is 1 or 2 |
| 32 | CD28 hinge | IEVMYPPPYLDNEKSNGTIIHVKGKHLCPSPLFPGPSKP |
| 33 | CD8 hinge | SHFVPVFLPAKPTTTPAPRPPTPAPTIASQPLSLRPEACRPAAGGAVH TRGLDFACD |

(continued)

| SEQ ID NO | Remark | Sequence |
|---|---|---|
| 34 | CD28 TM domain | FWVLVVVGGVLACYSLLVTVAFIIFWV |
| 35 | CD8 TM domain | IYIWAPLAGTCGVLLLSLVITLYC |
| 36 | 4-1-BB co-stimulation | KRGRKKLLYIFKQPFMRPVQTTQEEDGCSCRFPEEEEGGCEL |
| 37 | CD28 co-stimulation | RSKRSRLLHSDYMNMTPRRPGPTRKHYQPYAPPRDFAAYRS |
| 38 | OX40 co-stimulation | RRDQRLPPDAHKPPGGGSFRTPIQEEQADAHSTLAKI |
| 39 | CD3ζ | RVKFSRSADAPAYQQGQNQLYNELNLGRREEYDVLDKRRGRDPE MGGKPQRRKNPQEGLYNELQKDKMAEAYSEIGMKGERRRGKGHD GLYQGLSTATKDTYDALHMQALPPR |
| 40 | RFP | MASSEDVIKEFMRFKVRMEGSVNGHEFEIEGEGEGRPYEGTQTAKL KVTKGGPLPFAWDILSPQFQYGSKAYVKHPADIPDYLKLSFPEGFK WERVMNFEDGGVVTVTQDSSLQDGEFIYKVKLRGTNFPSDGPVMQ KKTMGWEASTERMYPEDGALKGEIKMRLKLKDGGHYDAEVKTTY MAKKPVQLPGAYKTDIKLDITSHNEDYTIVEQYERAEGRHSTGA |
| 41 | IRES DNA | CCACCTACATGGGGATCCCTCCCCCCCCCCTAACGTTACTGGCC GAAGCCGCTTGGAATAAGGCCGGTGTGCGTTTGTCTATATGTTA TTTTCCACCATATTGCCGTCTTTTGGCAATGTGAGGGCCCGGAAA CCTGGCCCTGTCTTCTTGACGAGCATTCCTAGGGGTCTTTCCCCT CTCGCCAAAGGAATGCAAGGTCTGTTGAATGTCGTGAAGGAAGC AGTTCCTCTGGAAGCTTCTTGAAGACAAACAACGTCTGTAGCGA CCCTTTGCAGGCAGCGGAACCCCCCACCTGGCGACAGGTGCCTC TGCGGCCAAAAGCCACGTGTATAAGATACACCTGCAAAGGCGG CACAACCCCAGTGCCACGTTGTGAGTTGGATAGTTGTGGAAAGA GTCAAATGGCTCTCCTCAAGCGTATTCAACAAGGGGCTGAAGGA TGCCCAGAAGGTACCCCATTGTATGGGATCTGATCTGGGGCCTC GGTGCACATGCTTTACATGTGTTTAGTCGAGGTTAAAAAAACGT CTAGGCCCCCCGAACCACGGGGACGTGGTTTTCCTTTGAAAAAC ACGATAATACCATGACC |
| 42 | H3L3/PP6373 VH CDR1 | GVTFSEAWMD |
| 43 | H3L3/PP6373 VH CDR2 | EIRDKPNSYVTYYAESVKG |
| 44 | H3L3/PP6373 VH CDR3 | AMDY |
| 45 | H3L3/PP6373 VL CDR1 | KSSQSLLYSNGKTYLN |
| 46 | H3L3/PP6373 VL CDR2 | QVSKLDP |
| 47 | H3L3/PP6373 VL CDR3 | MQGSSLPWT |

(continued)

| SEQ ID NO | Remark | Sequence |
|---|---|---|
| 48 | CAR-C31 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGGGGSDIVMTQTPLSLSVTIG QPASISCKSSQSLLYSNGKTYLNWLQQRPGQSPKRLIYQVSKLDPGI PDRFSGSGSETDFTLKISRVEAEDLGIYYCMQGSSLPWTFGGGTKLE IKASSHFVPVFLPAKPTTTPAPRPPTPAPTIASQPLSLRPEACRPAAG GAVHTRGLDFACDFWVLVVVGGVLACYSLLVTVAFIIFWVRSKRS RLLHSDYMNMTPRRPGPTRKHYQPYAPPRDFAAYRSRVKFSRSAD APAYQQGQNQLYNELNLGRREEYDVLDKRRGRDPEMGGKPQRRK NPQEGLYNELQKDKMAEAYSEIGMKGERRRGKGHDGLYQGLSTA TKDTYDALHMQALPPRGSGATNFSLLKQAGDVEENPGPMEDFNM ESDSFEDFWKGEDLSNYSYSSTLPPFLLDAAPCEPESLEINKYFVVII YALVFLLSLLGNSLVMLVILYSRVGRSVTDVYLLNLALADLLFALT LPIWAASKVNGWIFGTFLCKVVSLLKEVNFYSGILLLACISVDRYLA IVHATRTLTQKRYLVKFICLSIWGLSLLLALPVLLFRRTVYSSNVSP ACYEDMGNNTANWRMLLRILPQSFGFIVPLLIMLFCYGFTLRTLFK AHMGQKHRAMRVIFAVVLIFLLCWLPYNLVLLADTLMRTQVIQET CERRNHIDRALDATEILGILHSCLNPLIYAFIGQKFRHGLLKILAIHGL ISKDSLPKDSRPSFVGSSSGHTSTTL |
| 49 | CAR-C32 | EVKFEESGGGLVQPGGSIKLSCAASGVTFSEAWMDWVRQSPEKGL EWVAEIRDKPNSYVTYYAESVKGRFTISRDDSKSRVYLQMNNLRT EDTGIYYCTGAMDYWGQGTSVTVSSGGGGSDIVMTQTPLSLSVTIG QPASISCKSSQSLLYSNGKTYLNWLQQRPGQSPKRLIYQVSKLDPGI PDRFSGSGSETDFTLKISRVEAEDLGIYYCMQGSSLPWTFGGGTKLE IKASSHFVPVFLPAKPTTTPAPRPPTPAPTIASQPLSLRPEACRPAAG GAVHTRGLDFACDFWVLVVVGGVLACYSLLVTVAFIIFWVRSKRS RLLHSDYMNMTPRRPGPTRKHYQPYAPPRDFAAYRSRVKFSRSAD APAYQQGQNQLYNELNLGRREEYDVLDKRRGRDPEMGGKPQRRK NPQEGLYNELQKDKMAEAYSEIGMKGERRRGKGHDGLYQGLSTA TKDTYDALHMQALPPRGSGATNFSLLKQAGDVEENPGPMCHQQL VISWFSLVFLASPLVAIWELKKDVYVVELDWYPDAPGEMVVLTCD TPEEDGITWTLDQSSEVLGSGKTLTIQVKEFGDAGQYTCHKGGEVL SHSLLLLHKKEDGIWSTDILKDQKEPKNKTFLRCEAKNYSGRFTCW WLTTISTDLTFSVKSSRGSSDPQGVTCGAATLSAERVRGDNKEYEY SVECQEDSACPAAEESLPIEVMVDAVHKLKYENYTSSFFIRDIIKPDP PKNLQLKPLKNSRQVEVSWEYPDTWSTPHSYFSLTFCVQVQGKSK REKKDRVFTDKTSATVICRKNASISVRAQDRYYSSSWSEWASVPCS |
| 50 | P40 | MCHQQLVISWFSLVFLASPLVAIWELKKDVYVVELDWYPDAPGEM VVLTCDTPEEDGITWTLDQSSEVLGSGKTLTIQVKEFGDAGQYTCH KGGEVLSHSLLLLHKKEDGIWSTDILKDQKEPKNKTFLRCEAKNYS GRFTCWWLTTISTDLTFSVKSSRGSSDPQGVTCGAATLSAERVRGD NKEYEYSVECQEDSACPAAEESLPIEVMVDAVHKLKYENYTSSFFI RDIIKPDPPKNLQLKPLKNSRQVEVSWEYPDTWSTPHSYFSLTFCVQ VQGKSKREKKDRVFTDKTSATVICRKNASISVRAQDRYYSSSWSE WASVPCS* |
| 51 | P2A | GSGATNFSLLKQAGDVEENPGP |
| 52 | T2A | GSGEGRGSLLTCGDVEENPGP |

(continued)

| SEQ ID NO | Remark | Sequence |
|---|---|---|
| 53 | CXCR2 | MEDFNMESDSFEDFWKGEDLSNYSYSSTLPPFLLDAAPCEPESLEIN KYFVVIIYALVFLLSLLGNSLVMLVILYSRVGRSVTDVYLLNLALA DLLFALTLPIWAASKVNGWIFGTFLCKVVSLLKEVNFYSGILLLACI SVDRYLAIVHATRTLTQKRYLVKFICLSIWGLSLLLALPVLLFRRTV YSSNVSPACYEDMGNNTANWRMLLRILPQSFGFIVPLLIMLFCYGF TLRTLFKAHMGQKHRAMRVIFAVVLIFLLCWLPYNLVLLADTLMR TQVIQETCERRNHIDRALDATEILGILHSCLNPLIYAFIGQKFRHGLL KILAIHGLISKDSLPKDSRPSFVGSSSGHTSTTL |

## Example 1

**Expressions of H3L3-CAR and PP6373-CAR**

Anti-CD24 antibodies react with a broad spectrum of cancer cells and can be used to produce chimeric antigen receptors to endow T cells with the anticancer activity. In one embodiment, the antigen-binding fragments of CD24 were inserted into CAR-T vectors known in the art. These antigen-binding fragments include but are not limited to, for example, PP6373/H3L3 single-stranded Fv sequences (the heavy chain variable region can be as set forth in SEQ ID NO: 24, and the light chain variable region can be as set forth in SEQ ID NO: 26; or, the heavy chain variable region can be as set forth in SEQ ID NO: 23, and the light chain variable region can be as set forth in SEQ ID NO: 25), or other single-stranded anti-CD24 mAbs (alphaCD24SC). Then, constructs were inserted into gene vectors known in the art, including those derived from retroviruses, lentiviruses, adeno-associated viruses, or adenoviral vectors.

(I) Preparation of T Lymphocytes

PBMCs (Day 0) from healthy donors were stimulated for 24 h with anti-human CD3, anti-human CD28, and recombinant human fibronectin (RetroNectin, RN). Activated T cells were mocked (control T) or infected with lentivirus carrying CD24-CAR (Day 1). On Days 3 and 5, the culture medium was supplemented to carry out enlarged cultivation on the cells. On Day 7, the cells were stained with CD24-FITC, and FITC and RFP signals were detected by flow cytometry to identify the expression of antigen-binding regions on the surfaces of T cells.

(II) *In Vitro* Cytotoxic Test

In order to test the antitumor activity of CAR-T, the control T cells or CD24 CAR-T cells were co-cultured overnight with tumor cells that highly express target molecules CD24 and have luciferase activity. The lysis of tumor cells was measured by the luciferase detection kit (Yeasen) and calculated using the following formula:

$$\text{Lysis } \% = 100\% - \text{co-culture signal/tumor cell signal}.$$

(III) Cytokine Assay

In order to evaluate the safety of CAR-T, we measured the expression levels of interferon-gamma (IFN-γ) and interleukin-2 (IL-2) in the co-culture supernatant of CAR-T cells and tumor cells by the ELISA kit (Invitrogen).

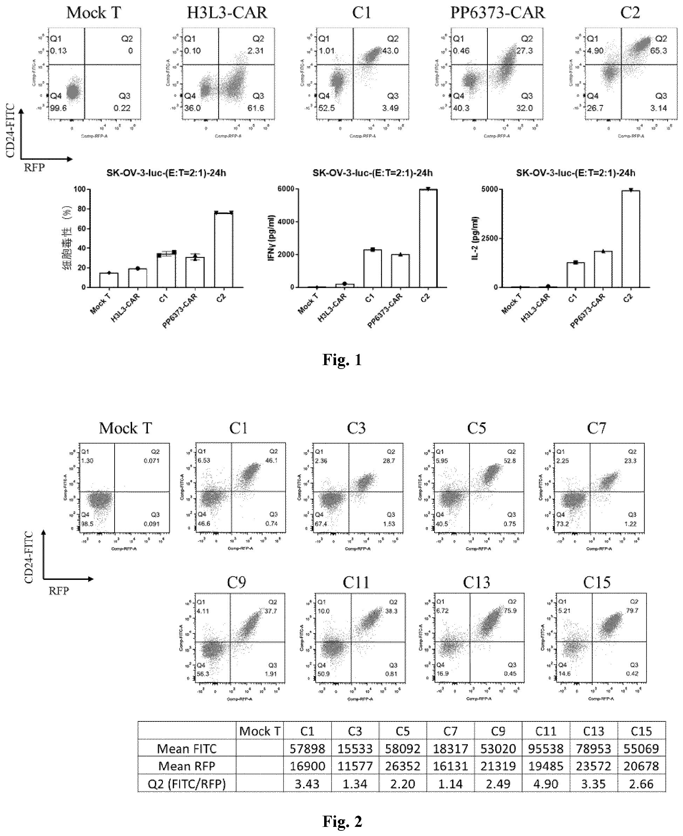

First, the structures of H3L3-CAR and PP6373-CAR were optimized by removing the first five amino acids in 4-1BB domain, to obtain C1 and C2; respectively. As shown in Fig. 1, the binding capacity of the antigen-binding regions of C1 and C2 on the surface of T cells was significantly increased. C1 and C2 also have higher antitumor activity and cytokine expression levels. The above results showed that compared with the previous H3L3-CAR and PP6373-CAR, the structurally optimized C1 and C2 could express the CAR structures more efficiently, and have stronger anti-tumor ability and higher cytokine secretion levels.

## Example 2

**Structural Optimization of CD24-CAR**

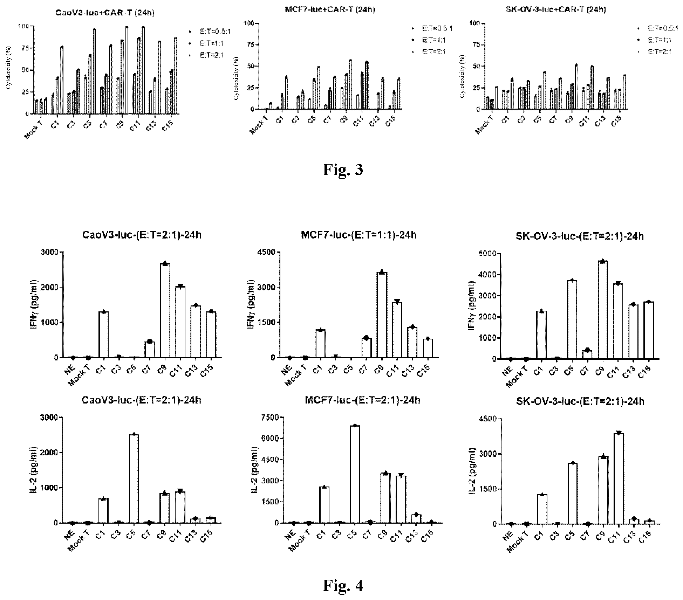

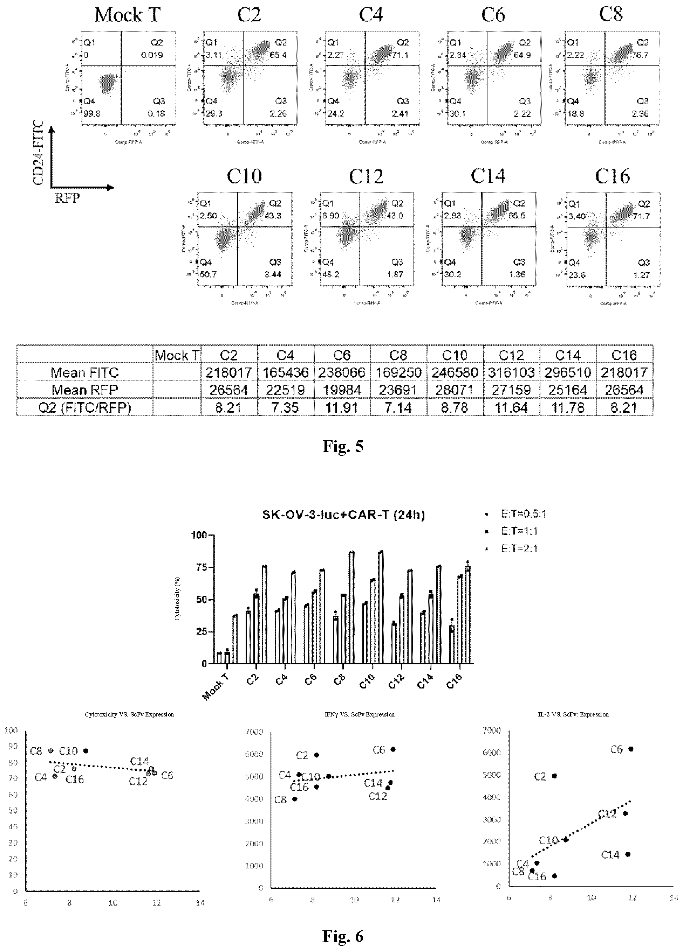

CAR structures such as C3, C5, C7, C9, C11, C13 and C15 were derived by adjusting the hinge region, transmembrane region and costimulatory domain of the CAR structure on the basis of C1 (Fig. 2). CAR structures such as C4, C6, C8, C10, C12, C14 and C16 were derived by adjusting the hinge region, transmembrane region and costimulatory domain of the CAR structure accordingly on the basis of C2 (Fig. 5). Co-culture results showed that C5, C8, C9, C10, and C11 had better antitumor activity (Fig. 3 and Fig. 6).

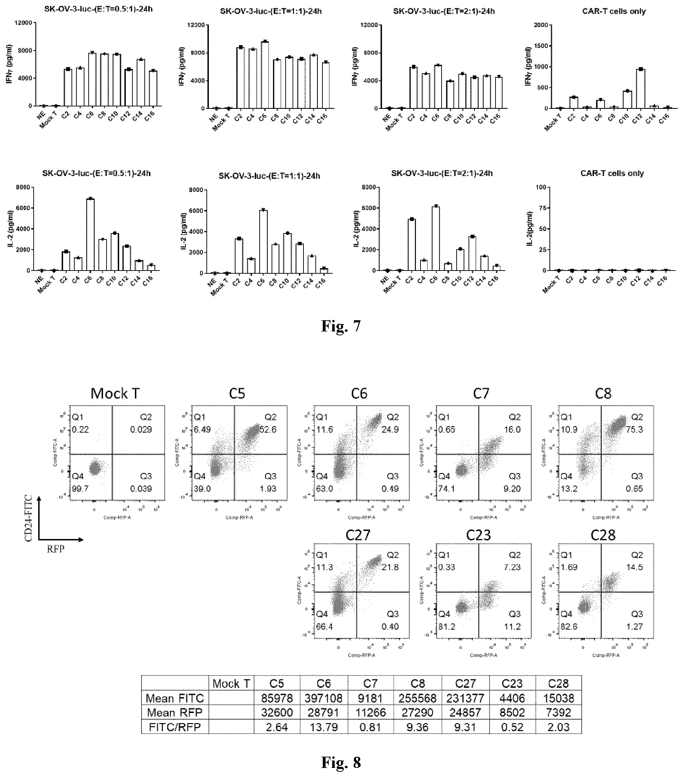

Based on further detection of cytokine secretion, it was found that C7/C8 produced IFN-γ and IL-2 at a lower level (Fig. 4, Fig. 7), indicating that the CAR structure of C7/C8 (hinge region: CD8, transmembrane region: CD28TM, and costimulatory domain: CD28) had better anti-tumor activity and better safety. It also indicated that in CD24-CAR, the second-generation CAR structure could achieve greater *in vitro* cytotoxic efficacy than that of the third-generation CAR.

**Example 3**

**Optimization of Linker in CD24-CAR Structure**

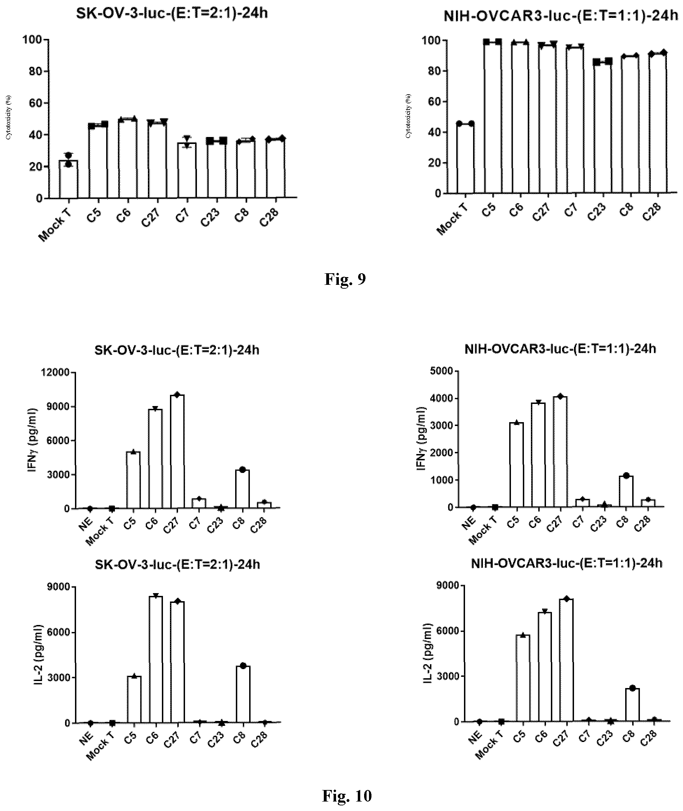

In order to further optimize the CAR structure, the linkers between the heavy and light chains of the C6 and C8 antibodies were adjusted, the amino acid sequence was shortened from (GGGGS)3 to GGGGS to obtain C27 and C28, and the C7 transmembrane region CD28TM was changed to CD8TM to obtain C23 (Fig. 8). By comparing the *in vitro* cytotoxic efficacy and cytokine secretion levels of these CAR structures, it was found that there was almost no difference in the *in vitro* cytotoxic efficacy for CAR-T cells (Fig. 9), but C28 with the shortened linker significantly reduced the cytokine secretion level (Fig. 10).

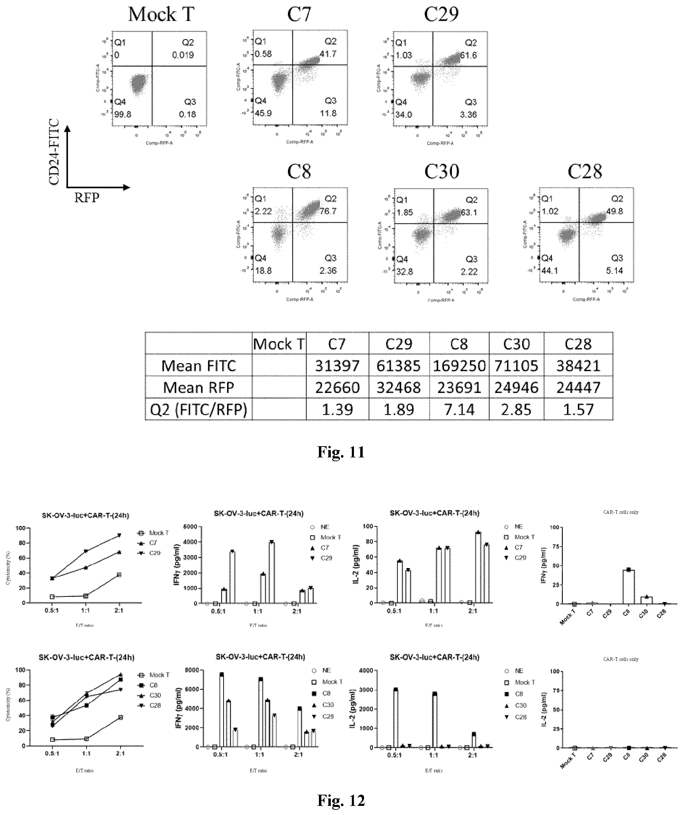

In order to further explore the effect of linker length on CAR function, the linker of C7 was extended from (GGGGS)3 to (GGGGS)4 to obtain C29; meanwhile, the linker of C8 was shortened from (GGGGS)3 to (GGGGS)2 to obtain C30 (Fig. 11). Based on *in vitro* experiments, we found that compared with C7, C29 with the extended linker had a significant increase in cytotoxic efficacy and IFN-γ secretion levels (Fig. 12). By comparing C8, C28 and C30, we also found that there was a positive correlation between the linker length and the IFN-γ secretion level. By comparing C8, C28 and C30, we found that there was a positive correlation between the linker length and the secretion levels of IFN-γ and IL-2, and shortening the linker length had little effect on the cytotoxic efficacy of CAR-T cells. The above results showed that we could optimize the anti-tumor effect and cytokine secretion level of CAR-T cells by modifying the linker length, and in case of high antigen-target affinity, shortening the linker could maintain the cytotoxic efficacy of CAR-T cells while significantly reduce the production of cytokines. Therefore, C28 was more effective in avoiding the risk of cytokine storm during treatment.

**Example 4**

**Functional Optimization of CD24-CAR**

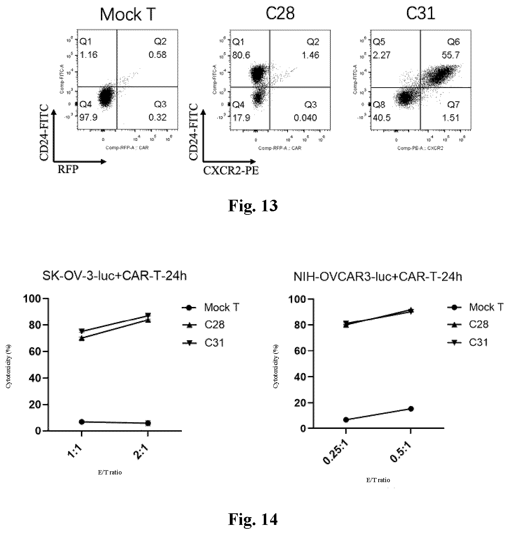

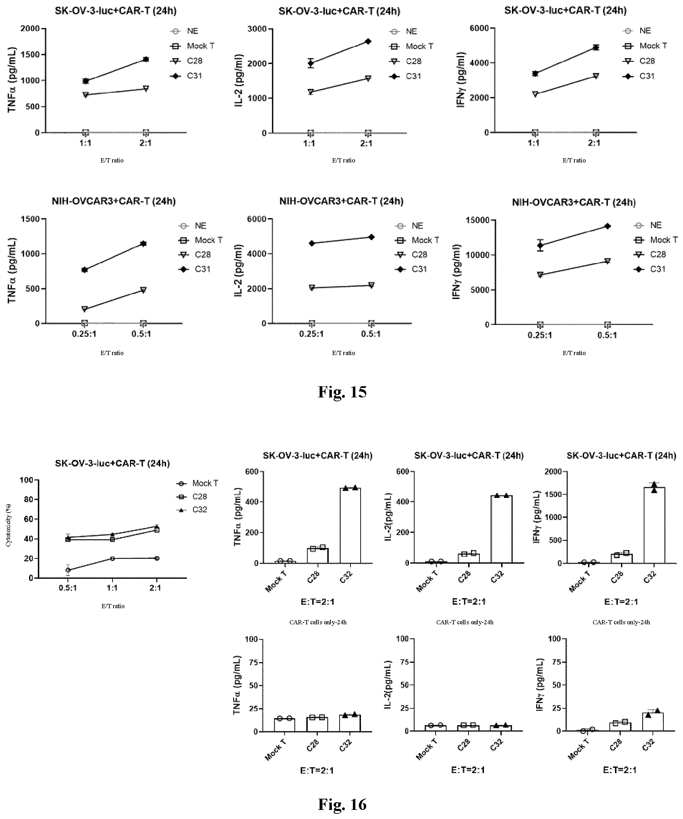

In order to enhance the chemotaxis capacity of CAR-T cells and the persistence of cytokine IFN-γ secretion, the chemokine receptor CXCR2 and cytokine IL-12β were respectively linked to the CD3ζ 3'-terminal of the C28 CAR sequence via P2A linkers, to obtain C31 and C32 (Fig. 13). Based on *in vitro* experiments, we found that compared with C28, C31 expressing the chemokine receptor CXCR2 showed significantly increased cytotoxic efficacy for SK-OV-3 target cells, and the concentration of IFN-γ, TNF-α, IL-2 and other cytokines produced by killing target cells SK-OV-3 and NIH-OVCAR3 also increased significantly (Fig. 14 and Fig. 15). By comparing C28 and C32, we found that there was no significant difference in the *in vitro* cytotoxic efficacy between C32 expressing IL-12β and C28, while the secretion levels of cytokines such as IFN-γ, TNF-α, and IL-2 increased significantly (Fig. 16). The above results showed that the expression of the chemokine receptor CXCR2 helped to improve the anti-tumor effect and cytokine secretion level of C28, meanwhile IL-12β could significantly enhance the cytokine release of C28.

**Example 5**

**Animal Model Test**

(I) Verification of *In Vivo* Functions of C8 and C28

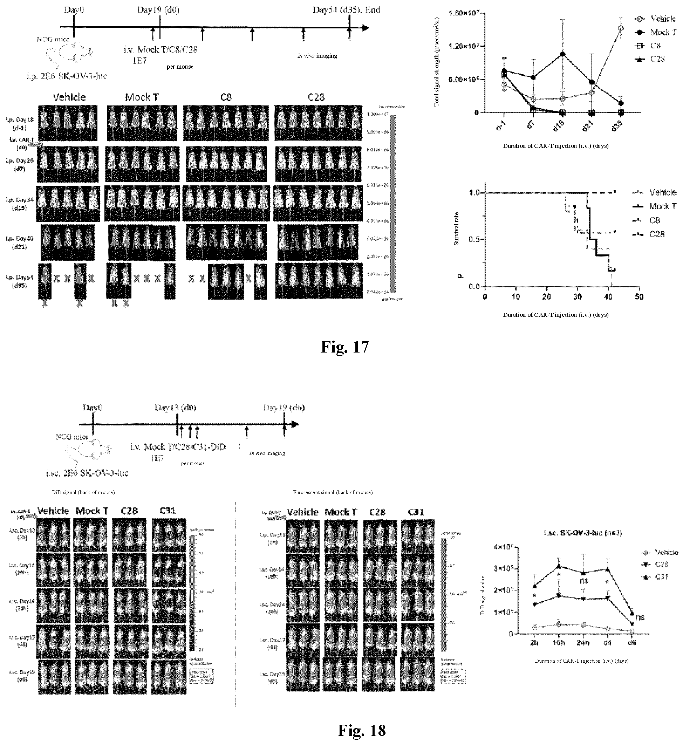

The purchased NCG immunodeficient mice of 4-5 weeks old were fed for one week in the SPF-grade animal rooms; then, each mouse was intraperitoneally injected with $2*10^6$ SK-OV-3-luc cells; 19 days after abdominal tumor formation, the intraperitoneal tumors of the mice were detected by an *in vivo* imager; the mice were grouped by tumor size, and injected at tail veins with $6*10^6$ C8, C28 and NC (i.e., Mock T) cells in each case and with normal saline as a blank control; and the mice were weighed weekly, and the distribution and size of tumors in these mice were monitored using the *in vivo* imager. As shown in Fig. 17, C8 and C28 substantially cleared tumor cells two weeks after reinfusion of CAR-T. The results showed that there was no significant difference between C8 and C28 in anti-tumor ability, and C28 had better *in vivo* safety.

(II) Verification of *In Vivo* Migration Ability of C28 and C31

The purchased NCG immunodeficient mice of 4-5 weeks old were fed for one week in the SPF-grade animal rooms; then, each mouse was subcutaneously injected on the back with $2*10^6$ SK-OV-3-luc cells; 13 days after tumor formation, the tumors on the backs of the mice were detected by an *in vivo* imager; the mice were grouped by tumor size, and injected at tail veins with $1*10^7$ C28, C31 and NC (i.e., Mock T) cells tagged with the fluorescent dye DiD in each case and with normal saline as a blank control; and the CAR-T and the distribution and size of tumors in these mice were monitored using the *in vivo* imager. As shown in Fig. 18, the DiD signals of C31 were significantly higher than those of C28 and control cells within 6 days after CAR-T cells reinfusion, indicating that C31 had stronger *in vivo* migration and survival ability.

(III) Comparison of *In Vivo* Functions of C28 and C31

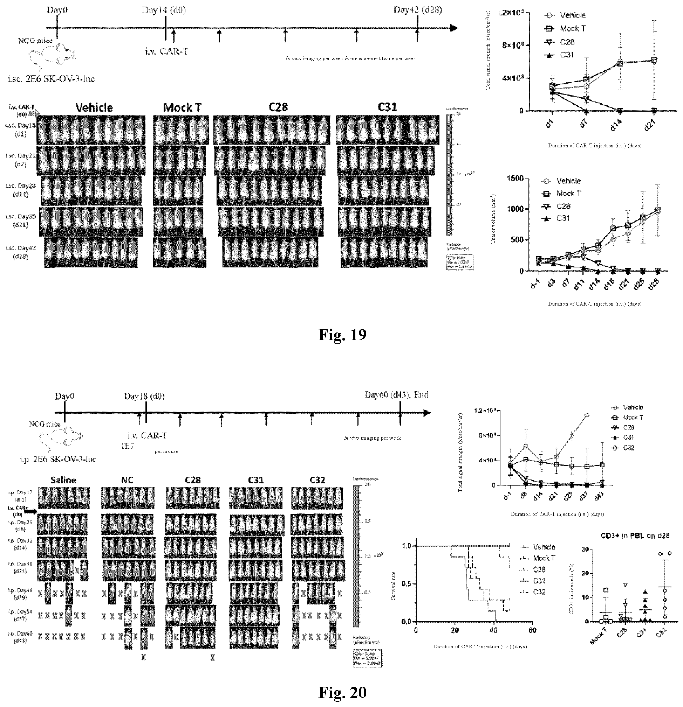

The purchased NCG immunodeficient mice of 4-5 weeks old were fed for one week in the SPF-grade animal rooms; then, each mouse was subcutaneously injected on the back with $2*10^6$ SK-OV-3-luc cells; 14 days after abdominal tumor formation, the intraperitoneal tumors of the mice were detected by an *in vivo* imager; the mice were grouped by tumor size, and injected at tail veins with $1*10^7$ C28, C31 and NC (i.e., Mock T) cells in each case and with normal saline as a blank control; and the mice were weighed weekly, and the distribution and size of tumors in these mice were monitored using the *in vivo* imager. As shown in Fig. 19, both C28 and C31 were able to clear tumor cells after reinfusion, C31 completely cleared the tumor cells in the first week after CAR-T cells reinfusion, and C28 was able to clear the tumor cells in the second week after reinfusion. There was no death of mice during the experiment, which also indicated better safety of C28 and C31.

(IV) Comparison of *In Vivo* Functions of C28, C31 and C32

The purchased NCG immunodeficient mice of 4-5 weeks old were fed for one week in the SPF-grade animal rooms; then, each mouse was intraperitoneally injected with $2*10^6$ SK-OV-3-luc cells; 18 days after abdominal tumor formation, the intraperitoneal tumors of the mice were detected by an *in vivo* imager; the mice were grouped by tumor size, and injected at tail veins with $1*10^7$ C28, C31, C32 and NC (i.e., Mock T) cells in each case and with normal saline as a blank control; and the mice were weighed weekly, and the distribution and size of tumors in these mice were monitored using the *in vivo* imager. As shown in Fig. 20, both C31 and C32 substantially cleared tumor cells in the first week after CAR-T infusion, and completely removed the tumors in the second week after reinfusion. However, the death of mice occurred 4 weeks after T cell reinfusion with C32, and the reason for the death of mice might be the fact that C32 had good amplification capacity, and a large number of T cells appeared in the peripheral blood of NCG mice to result in GVHD to eventually cause the death of mice. Furthermore, C28 could substantially clear the tumor cells in the third week after reinfusion, indicating good tumor removal effect of C28. These results showed that C31 had better tumor removal capacity and higher *in vivo* safety.

In summary, the present application exemplifies the great potential of the antibody CD24 CAR-T based on the present application in cancer treatment. It has high safety and efficacy in both *in vivo* and *in vitro* experiments, without the off-target risk of killing normal PBMCs.

The foregoing detailed description is provided by way of explanation and examples, and is not intended to limit the scope of the appended claims. Various changes of the embodiments listed in the present application until now would be obvious to those of ordinary skills in the art, and should be kept within the scope of the appended claims and equivalents thereof.

**Claims**

1. A group of improved antigen-binding protein G1, wherein the G1 is capable of binding to CD24, and the enhanced effect of cells expressing said G1 on the killing ability of CD24-positive tumor cells, or the ability of cells expressing said G1 to express cytokines upon stimulation by CD24-positive tumor cells, is higher than that of cells expressing the existing CD24 antigen-binding proteins.

2. The antigen-binding protein G1 of claim 1, wherein said cytokines comprise IFN-γ, and/or IL-2.

3. A group of further improved antigen-binding protein G2 based on said G1 of any one of claims 1 to 2, wherein said G2 is capable of binding to CD24, G2-expressing cells maintain a capacity to kill said CD24-positive tumor cells, and an ability of expressing cytokines after stimulation by said CD24-positive tumor cells is at least about 50% lower than that of cells expressing G1.

4. A group of further improved antigen-binding protein G3 based on said G1 of any one of claims 1 to 2 or based on said G2 of claim 3 for use in expression in cells, wherein the G3-expressing cells also express chemokine receptors, and preferably, said chemokine receptors comprise CXCR2.

5. A group of further improved antigen-binding protein G3 based on said G1 of any one of claims 1 to 2 or based on said G2 of claim 3 for use in expression in cells, wherein the G3-expressing cells also express cytokines, and preferably, said cytokines comprise IL-12β.

6. The antigen-binding protein of any one of claims 1 to 5, wherein the antigen-binding fragment comprises a light chain variable region and a heavy chain variable region, with a linker between said light chain variable region and said heavy chain variable region, said linker having a length of 10 amino acids or less.

7. The antigen-binding protein of claim 6, wherein said linker has a length of 5 amino acids or less.

8. The antigen-binding protein of any one of claims 1 to 7, wherein said antigen-binding protein comprises H-CDRs 1 to 3 in said heavy chain variable region as set forth in SEQ ID NO: 23 or 24, and L-CDRs 1 to 3 in said light chain variable region as set forth in SEQ ID NO: 25 or 26.

9. The antigen-binding protein of any one of claims 1 to 8, wherein said antigen-binding protein comprises H-CDRs 1 to 3 in said heavy chain variable region as set forth in SEQ ID NOs: 42 to 44, and L-CDRs 1 to 3 in said light chain variable region as set forth in SEQ ID NOs: 45 to 47.

10. The antigen-binding protein of any one of claims 1 to 9, wherein the signal peptide comprises a sequence as set forth in SEQ ID NO: 22.

11. The antigen-binding protein of any one of claims 1 to 10, wherein said antigen-binding protein further comprises a hinge domain, which is directly or indirectly linked to said antigen-binding domain.

12. The antigen-binding protein of claim 11, wherein said hinge domain comprises a hinge domain of CD8 or CD28, or of a functional fragment thereof.

13. The antigen-binding protein of any one of claims 11 to 12, wherein said hinge domain comprises a sequence as set forth in SEQ ID NO: 32 or 33.

14. The antigen-binding protein of any one of claims 1 to 13, wherein said antigen-binding protein further comprises a transmembrane domain, which is directly or indirectly linked to said hinge domain.

15. The antigen-binding protein of claim 14, wherein said transmembrane domain comprises a transmembrane domain of CD8 or CD28, or of a functional fragment thereof.

16. The antigen-binding protein of any one of claims 14 to 15, wherein said transmembrane domain comprises a sequence as set forth in SEQ ID NO: 34 or 35.

17. The antigen-binding protein of any one of claims 1 to 16, wherein said antigen-binding protein further comprises an intracellular domain, which is directly or indirectly linked to said transmembrane domain.

18. The antigen-binding protein of claim 17, wherein said intracellular domain comprises a costimulatory domain, which comprises a costimulatory domain of 41-BB, CD28, or OX40, or of a functional fragment thereof.

19. The antigen-binding protein of claim 18, wherein said antigen-binding protein comprises one or more of said costimulatory domains.

20. The antigen-binding protein of any one of claims 18 to 19, wherein said costimulatory domain comprises a sequence as set forth in SEQ ID NO: 36, 37, or 38.

21. The antigen-binding protein of any one of claims 1 to 20, wherein said intracellular domain comprises a signaling domain, which comprises a signaling domain of CD3ζ or a functional segment thereof.

22. The antigen-binding protein of claim 21, wherein said intracellular signaling domain comprises a sequence as set forth in SEQ ID NO: 39.

23. The antigen-binding protein of any one of claims 1 to 22, wherein said antigen-binding protein comprises a chimeric antigen receptor.

24. The antigen-binding protein of claims 1 to 23, wherein said antigen-binding protein is selected from a sequence as set forth in any one of SEQ ID NOs: 1 to 21, 48 and 49.

25. A polypeptide, comprising said antigen-binding protein of any one of claims 1 to 24.

26. The polypeptide of claim 25, wherein said polypeptide further comprises a tagged protein, which comprises a sequence as set forth in SEQ ID NO: 40.

27. The polypeptide of claim 26, wherein said tagged protein and said antigen-binding protein of said polypeptide are linked via a sequence as set forth in SEQ ID NO: 41.

28. A nucleic acid, encoding said antigen-binding protein of any one of claims 1 to 24 and/or said polypeptide of any one of claims 25 to 27.

29. A vector, comprising said nucleic acid of claim 28.

30. A cell, comprising said antigen-binding protein of any one of claims 1 to 24, said polypeptide of any one of claims 25 to 27, said nucleic acid of claim 28, and/or said vector of claim 29.

31. The cell of claim 30, wherein said cell comprises an immune cell.

32. The cell of any one of claims 30 to 31, wherein said cell comprises an NK cell and/or a T cell.

33. A preparation method for said antigen-binding protein of any one of claims 1 to 24 and/or said polypeptide of any one of claims 25 to 27, wherein said preparation method comprises culturing said cell of any one of claims 30 to 32 under conditions allowing expression of said antigen-binding protein and/or said polypeptide.

34. A composition, comprising said antigen-binding protein of any one of claims 1 to 24, said polypeptide of any one of claims 25 to 27, said nucleic acid of claim 28, said vector of claim 29, and/or said cell of any one of claims 30 to 32, and optionally a pharmaceutically acceptable carri er.

35. A kit, comprising said antigen-binding protein of any one of claims 1 to 24, said polypeptide of any one of claims 25 to 27, said nucleic acid of claim 28, said vector of claim 29, said cell of any one of claims 30 to 32, and/or said composition of claim 34.

36. A method for stimulating an immune response, comprising administering said antigen-binding protein of any one of claims 1 to 24, said polypeptide of any one of claims 25 to 27, said nucleic acid of claim 28, said vector of claim 29, said cell of any one of claims 30 to 32, said composition of claim 34 and/or said kit of claim 35.

37. The use of said antigen-binding protein of any one of claims 1 to 24, said polypeptide of any one of claims 25 to 27, said nucleic acid of claim 28, said vector of claim 29, said cell of any one of claims 30 to 32, said composition of claim 34 and/or said kit of claim 35 in the preparation of a medicament, said medicament is used for preventing, alleviating and/or treating a tumor.

38. The use of claim 37, wherein said tumor comprise solid tumor and/or hematologic tumor.

**39.** The use of any one of claims 37 to 38, wherein said tumor is selected from the group consisting of: lung cancer, breast cancer, liver cancer, brain cancer, cervical cancer, ovarian cancer, kidney cancer, testicular cancer, prostate cancer, and neuroblastoma.

Fig. 1

| | Mock T | C1 | C3 | C5 | C7 | C9 | C11 | C13 | C15 |
|---|---|---|---|---|---|---|---|---|---|
| Mean FITC | | 57898 | 15533 | 58092 | 18317 | 53020 | 95538 | 78953 | 55069 |
| Mean RFP | | 16900 | 11577 | 26352 | 16131 | 21319 | 19485 | 23572 | 20678 |
| Q2 (FITC/RFP) | | 3.43 | 1.34 | 2.20 | 1.14 | 2.49 | 4.90 | 3.35 | 2.66 |

Fig. 2

**Fig. 3**

**Fig. 4**

| | Mock T | C2 | C4 | C6 | C8 | C10 | C12 | C14 | C16 |
|---|---|---|---|---|---|---|---|---|---|
| Mean FITC | | 218017 | 165436 | 238066 | 169250 | 246580 | 316103 | 296510 | 218017 |
| Mean RFP | | 26564 | 22519 | 19984 | 23691 | 28071 | 27159 | 25164 | 26564 |
| Q2 (FITC/RFP) | | 8.21 | 7.35 | 11.91 | 7.14 | 8.78 | 11.64 | 11.78 | 8.21 |

**Fig. 5**

**Fig. 6**

Fig. 7

| | Mock T | C5 | C6 | C7 | C8 | C27 | C23 | C28 |
|---|---|---|---|---|---|---|---|---|
| Mean FITC | | 85978 | 397108 | 9181 | 255568 | 231377 | 4406 | 15038 |
| Mean RFP | | 32600 | 28791 | 11266 | 27290 | 24857 | 8502 | 7392 |
| FITC/RFP | | 2.64 | 13.79 | 0.81 | 9.36 | 9.31 | 0.52 | 2.03 |

Fig. 8

**Fig. 9**

**Fig. 10**

|  | Mock T | C7 | C29 | C8 | C30 | C28 |
|---|---|---|---|---|---|---|
| Mean FITC |  | 31397 | 61385 | 169250 | 71105 | 38421 |
| Mean RFP |  | 22660 | 32468 | 23691 | 24946 | 24447 |
| Q2 (FITC/RFP) |  | 1.39 | 1.89 | 7.14 | 2.85 | 1.57 |

**Fig. 11**

**Fig. 12**

**Fig. 13**

**Fig. 14**

Fig. 15

Fig. 16

Fig. 17

Fig. 18

Fig. 19

Fig. 20

## INTERNATIONAL SEARCH REPORT

| International application No. |
|---|
| **PCT/CN2023/127598** |

| A. | CLASSIFICATION OF SUBJECT MATTER |
|---|---|

C07K19/00(2006.01)i; C12N15/13(2006.01)i; C12N15/62(2006.01)i; C12N5/10(2006.01)i; A61K 35/17(2015.01)i; A61P35/00(2006.01)i

According to International Patent Classification (IPC) or to both national classification and IPC

| B. | FIELDS SEARCHED |
|---|---|

Minimum documentation searched (classification system followed by classification symbols)

IPC:C07K,C12N,A61K,A61P

Documentation searched other than minimum documentation to the extent that such documents are included in the fields searched

Electronic data base consulted during the international search (name of data base and, where practicable, search terms used)

CNKI, CNABS, CNTXT, WPABSI, WPABSC, ENTXTC, ENTXT, ISI web of science and search terms: CD24, 结合, 抗原嵌合抗体, CAR, bind+, etc.; GENBANK, EMBL, 中国专利生物序列检索系统和检索的序列: SEQ ID NOs: 1-53, GENBANK, EMBL, China Patent Biological Sequence Search System and searched sequences: SEQ ID NOs: 1-53.

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 112424441 A (ONCOIMMUNE, INC. et al.) 26 February 2021 (2021-02-26) claims 1-32, figure 23, and embodiment 7 | 1-39 |
| X | CN 108373504 A (GRACELL BIOTECHNOLOGY (SHANGHAI) CO., LTD.) 07 August 2018 (2018-08-07) claims 1-10, and embodiments 12-14 | 1-7, 10-23, 25-39 |
| A | CN 108373504 A (GRACELL BIOTECHNOLOGY (SHANGHAI) CO., LTD.) 07 August 2018 (2018-08-07) claims 1-10, and embodiments 12-14 | 8, 9, 24 |
| A | CN 103819561 A (CHINA PHARMACEUTICAL UNIVERSITY) 28 May 2014 (2014-05-28) claims 1-15 | 1-39 |
| X | CN 112725283 A (WUHAN UNIVERSITY OF SCIENCE AND TECHNOLOGY) 30 April 2021 (2021-04-30) claims 1-8 | 1-7, 10-23, 25-39 |

☑ Further documents are listed in the continuation of Box C. ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **25 December 2023** | **05 January 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/127598**

### C. DOCUMENTS CONSIDERED TO BE RELEVANT

| Category* | Citation of document, with indication, where appropriate, of the relevant passages | Relevant to claim No. |
|---|---|---|
| A | CN 112725283 A (WUHAN UNIVERSITY OF SCIENCE AND TECHNOLOGY) 30 April 2021 (2021-04-30)<br>claims 1-8 | 8, 9, 24 |
| X | WO 2019111250 A1 (MEDICAL RES INFRASTRUCTURE & HEALTH SERVICES FUND TEL AVIV MEDICAL CT et al.) 13 June 2019 (2019-06-13)<br>claims 1-67 | 1-7, 10-23, 25-39 |
| A | WO 2019111250 A1 (MEDICAL RES INFRASTRUCTURE & HEALTH SERVICES FUND TEL AVIV MEDICAL CT et al.) 13 June 2019 (2019-06-13)<br>claims 1-67 | 8, 9, 24 |

Form PCT/ISA/210 (second sheet) (July 2022)

**INTERNATIONAL SEARCH REPORT**

International application No.

**PCT/CN2023/127598**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐    accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2023/127598** |

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **36**
   because they relate to subject matter not required to be searched by this Authority, namely:

   PCT Rule 39.1(iv) – methods for treatment of the human or animal body by surgery or therapy.

   The search for claim 36 is made on the basis of the reasonably expected subject matter, i.e., the use of the antigen-binding protein of any one of claims 1-24, the polypeptide of any one of claims 25-27, the nucleic acid of claim 28, the carrier of claim 29, the cell of any one of claims 30-32, the composition of claim 34 and/or the kit of claim 35 in the preparation of a drug for stimulating an immune response.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/127598**

| Patent document cited in search report | | | Publication date (day/month/year) | Patent family member(s) | | | Publication date (day/month/year) |
|---|---|---|---|---|---|---|---|
| CN | 112424441 | A | 26 February 2021 | JP | 2023123502 | A | 05 September 2023 |
| | | | | EA | 202092306 | A1 | 19 February 2021 |
| | | | | BR | 112020022482 | A2 | 09 February 2021 |
| | | | | ZA | 202006647 | B | 26 January 2022 |
| | | | | EP | 3813877 | A1 | 05 May 2021 |
| | | | | EP | 3813877 | A4 | 11 May 2022 |
| | | | | WO | 2019222082 | A1 | 21 November 2019 |
| | | | | CA | 3099554 | A1 | 21 November 2019 |
| | | | | US | 2021214458 | A1 | 15 July 2021 |
| | | | | MX | 2020012091 | A | 28 April 2021 |
| | | | | JP | 2021526509 | A | 07 October 2021 |
| | | | | JP | 7294758 | B2 | 20 June 2023 |
| | | | | SG | 11202010589 | YA | 27 November 2020 |
| | | | | AU | 2019269361 | A1 | 19 November 2020 |
| | | | | KR | 20210008487 | A | 22 January 2021 |
| | | | | TW | 202012434 | A | 01 April 2020 |
| CN | 108373504 | A | 07 August 2018 | None | | | |
| CN | 103819561 | A | 28 May 2014 | None | | | |
| CN | 112725283 | A | 30 April 2021 | None | | | |
| WO | 2019111250 | A1 | 13 June 2019 | EP | 3720882 | A1 | 14 October 2020 |
| | | | | EP | 3720882 | A4 | 27 October 2021 |
| | | | | US | 2021300986 | A1 | 30 September 2021 |
| | | | | IL | 275040 | A | 30 July 2020 |

Form PCT/ISA/210 (patent family annex) (July 2022)